# EUROPEAN PATENT SPECIFICATION

(11) **EP 0 448 704 B1**
(45) Date of publication and mention of the grant of the patent: **17.06.1998**
(21) Application number: 90916655.5
(22) Date of filing: 15.10.1990
(51) Int. Cl.: C07K 14/51, C07K 17/02, C09H 1/02, A61K 38/17

(54) **OSTEOGENIC DEVICES**
OSTEOGENE VORRICHTUNGEN
DISPOSITIFS OSTEOGENES

(30) Priority: 17.10.1989 US 422699; 22.02.1990 US 483913; 20.08.1990 US 569920
(43) Date of publication of application: 02.10.1991
(73) Proprietor: STRYKER CORPORATION, Kalamazoo, Michigan 49003-4085 (US)
(72) Inventor: OPPERMANN, Hermann, Medway, MA 02053 (US); KUBERASAMPATH, Thangavel, Medway, MA 02053 (US); RUEGER, David, C., West Roxbury, MA 02132 (US); OZKAYNAK, Engin, Milford, MA 01757 (US); PANG, Roy, H., L., Medway, MA 02053 (US)
(74) Representative: Hutchins, Michael Richard
(86) International application number: US9005903
(87) International publication number: WO9105802

(56) References cited:
- EP-A- 0 128 041
- EP-A- 0 148 155
- EP-A- 0 169 001
- EP-A- 0 169 016
- EP-A- 0 182 483
- EP-A- 0 212 474
- EP-A- 0 230 647
- EP-A- 0 309 241
- WO-A-86/00526
- WO-A-88/00205
- WO-A-89/09605
- WO-A-89/09788
- WO-A-89/10409
- WO-A-90/11366
- US-A- 4 172 128
- US-A- 4 294 753
- US-A- 4 394 370
- US-A- 4 434 094
- US-A- 4 563 350
- US-A- 4 563 489
- US-A- 4 657 548
- US-A- 4 703 108
- US-A- 4 725 671
- US-A- 4 789 663
- US-A- 4 812 120
- US-A- 4 824 939
- US-A- 4 837 285
- US-A- 4 894 441
- PROC. NATL. ACAD. SCI. USA, vol. 86, June 1989, pages 4554-4558; K. LYONS et al.: "Vgr-1, a mammalian gene related to Xenopus Vg-1, is a member of the transforming growth factor beta gene superfamily"
- THE EMBO JOURNAL, vol. 9, no. 7, 1990, pages 2085-2093, Oxford University Press; E. ÖZKAYNAK et al.: "OP-1 cDNA encodes an osteogenic protein in the TGF- beta family"
- Proc. Natl. Acad. Sci., USA, Vol. 80, November 1983, (SAMPATH), "Homology of bone-inductive proteins from human, monkey, bovine and rat extracellular matrix" pages 6591-6595. See "Materials and Methods" for production of the matrix.
- Proc. Natl. Acad. Sci., Vol. 78, No. 12, USA, December 1981 (SAMPATH), "Dissociative extraction and reconstitution of extracellular matrix components involved in local bone differentiation". Pages 7599-7603. See page 7599 "Preparation of Demineralized Bone", See entire section.
- Collagen Research, Vol. 1/1981 A.H. REDDI "Cell Biology and Biochemistry of Endochondral Bone Development" pages 209-226.
- The Lancet, May 2, 1981, JULIE GLOWACKI "Application of the Biological Principle of Induced Osteogenesis for Craniofacial Defects" pages 959-963.
- Journal of Biomedical Materials Research, Vol. 19, 1985, A.H. REDDI "Implant- stimutlated interface reactions during collageous bone matrix-induced bone formation" pages 233-239.
- Clinical Orthopaedics and Related Research Number 187, July/August 1984, MARSHALL R. Urist "beta-tricalcium Phosphate Delivery System for Bone Morphogenetic Protein" pages 277-280.
- Biotechnology and Bioengineering. Vol. XXVI, 1984 JANIE M. strand "A Modified Matrix Perfusion-Micro-carrier Bead Cell Culture System. I Adaption of the Matrix Perfusion System for Growth of Human Foreskin Fibroblasts" pages 503- 507.
- Clinical Orthopaedics and Related Research, Number 232, July 1988 STEPHAN D. COOK "Hydroxyapatite-Coated Titanium for Orthopaedic Implant Applications" pages 225-243.
- Journal of Arthroplasty, Vol 2, No. 2, June 1987 Myron Spector "Historical Review of Porous Coated Implants" pages 163-177.
- Int. J. Orla Maxillofac. Surg. Vol. 17, 1988 J.R. DEATHERAGE "A review of matrix-induced osteogenesis with special reference to its potential use in craniofacial surgery" pages 395-399.
- J. Bone JoinstSurg. Vol. 70-B, 1988 Per Aspenberg "Failure of Bone Induction by Bone Matrix in Adult Monkeys" pages 625-627.

## Description

### Reference to Related Applications

This application is a continuation-in-part of copending U.S. application Serial No. 422,699 filed October 17, 1989 entitled "Osteogenic Devices," and U.S. application Serial No. 483,913, filed February 22, 1990, entitled "Bone Collagen Matrix for Implants."

### Backcground of the Invention

This invention relates to the subject matter of the claims.

Mammalian bone tissue is known to contain one or more proteinaceous materials, presumably active during growth and natural bone healing, which can induce a developmental cascade of cellular events resulting in endochondral bone formation. This active factor (or factors) has variously been referred to in the literature as bone morphogenetic or morphogenic protein, bone inductive protein, osteogenic protein, osteogenin, or osteoinductive protein.

The developmental cascade of bone differentiation consists of recruitment of mesenchymal cells, proliferation of progenitor cells, calcification of cartilage, vascular invasion, bone formation, remodeling, and finally marrow differentiation (Reddi (1981) Collagen Rel. Res. 1:209-226).

Though the precise mechanisms underlying these phenotypic transformations are unclear, it has been shown that the natural endochondral bone differentiation activity of bone matrix can be dissociatively extracted and reconstituted with inactive residual collagenous matrix to restore full bone induction activity (Sampath and Reddi (1981) Proc. Natl. Acad. Sci. USA 78:7599-7603). This provides an experimental method for assaying protein extracts for their ability to induce endochondral bone in vivo. Several species of mammals produce closely related protein as demonstrated by cross species implant experiments (Sampath and Reddi (1983) Proc. Natl. Acad. Sci. USA 80:6591-6595).

The potential utility of these proteins has been recognized widely. It is contemplated that the availability of the protein would revolutionize orthopedic medicine, certain types of plastic surgery, and various periodontal and craniofacial reconstructive procedures.

The observed properties of these protein fractions have induced an intense research effort in several laboratories directed to isolating and identifying the pure factor or factors responsible for osteogenic activity. The current state of the art of purification of osteogenic protein from mammalian bone is disclosed by Sampath et al. (1987) Proc. Natl. Acad. Sci. USA 80. Urist et al. (1984) Proc. Soc. Exp. Biol. Med. 173:194-199 disclose a human osteogenic protein fraction which was extracted from demineralized cortical bone by means of a calcium chloride-urea inorganic-organic solvent mixture, and retrieved by differential precipitation in guanidine-hydrochloride and preparative gel electrophoresis. The authors report that the protein fraction has an amino acid composition of an acidic polypeptide and a molecular weight in a range of 17-18 kD.

Urist et al. (1984) Proc. Natl. Acad. Sci. USA 81:371-375 disclose a bovine bone morphogenetic protein extract having the properties of an acidic polypeptide and a molecular weight of approximately 18 kD. The authors reported that the protein was present in a fraction separated by hydroxyapatite chromatography, and that it induced bone formation in mouse hindquarter muscle and bone regeneration in trephine defects in rat and dog skulls. Their method of obtaining the extract from bone results in ill-defined and impure preparations.

European Patent Application Serial No. 148,155, published October 7, 1985, purports to disclose osteogenic proteins derived from bovine, porcine, and human origin. One of the proteins, designated by the inventors as a P3 protein having a molecular weight of 22-24 kD, is said to have been purified to an essentially homogeneous state. This material is reported to induce bone formation when implanted into animals.

International Application No. PCT/087/01537, published January 14, 1988 (Int. Pub. No. WO88/00205), discloses an impure fraction from bovine bone which has bone induction qualities. The named applicants also disclose putative "bone inductive factors" produced by recombinant DNA techniques. Four DNA sequences were retrieved from human or bovine genomic or cDNA libraries and expressed in recombinant host cells. While the applicants stated that the expressed proteins may be bone morphogenic proteins, bone induction was not demonstrated, suggesting that the recombinant proteins are not osteogenic. The same group reported subsequently (Science, 242:1528, Dec. 1988) that three of the four factors induce cartilage formation, and postulate that bone formation activity "is due to a mixture of regulatory molecules" and that "bone formation is most likely controlled ... by the interaction of these molecules." Again, no bone induction was attributed to the products of expression of the cDNAs. See also Urist et al., EP0 212,474 entitled Bone Morphogenic Agents.

Wang et al. (1988) Proc. Nat. Acad. Sci. USA 85: 9484-9488, disclose the purification of a bovine bone morphogenetic protein from guanidine extracts of demineralized bone having cartilage and bone formation activity as a basic protein corresponding to a molecular weight of 30 kD determined from gel elution. Purification of the protein yielded proteins of 30, 18 and 16 kD which, upon separation, were inactive. In view of this result, the authors acknowledged that the exact identity of the active material had not been determined.

Wang et al. (1990) Proc. Nat. Acad. Sci. USA 87: 2220-2227 describes the expression and partial purification of one of the cDNA sequences described in PCT 87/01537. Consistent cartilage and/or bone formation with their protein requires a minimum of 600 ng of 50% pure material.

International Application No. PCT/89/04458 published April 19, 1990 (Int. Pub. No. WO90/003733), describes the purification and analysis of a family of osteogenic factors called "P3 OF 31-34". The protein family contains at least four proteins, which are characterized by peptide fragment sequences. The impure mixture P3 OF 31-34 is assayed for osteogenic activity. The activity of the individual proteins is neither assessed nor discussed.

It has been found that successful implantation of the osteogenic factors requires association of the proteins with a suitable carrier material capable of maintaining the proteins at an in vivo site of application. The carrier should be biocompatible, biodegradable and porous enough to allow cell infiltration. The insoluble collagen particles remaining after guanidine extraction and delipidation of pulverized bone generally have been found effective in allogenic implants in some species. However, studies have shown that while osteoinductive proteins are useful cross species, the collagenous bone matrix generally used for inducing endochondral bone formation is species specific (Sampath and Reddi (1983) Proc. Nat. Acad. Sci. USA 80:6591-6594). Demineralized, delipidated, extracted xenogenic bone matrix carriers implanted in vivo invariably fail to induce osteogenesis, presumably due to inhibitory or immunogenic components in the bone matrix. Even the use of allogenic bone matrix in osteogenic devices may not be sufficient for osteoinductive bone formation in many species. For example, allogenic, subcutaneous implants of demineralized, delipidated monkey bone matrix is reported not to induce bone formation in the monkey. (Asperberg et al. (1988) J. Bone Joint Surg. (Br) 70-B:625-627).

U.S. 4,563,350, published January 7, 1986, discloses the use of trypsinized bovine bone matrix as a xenogenic matrix to effect osteogenic activity when implanted with extracted, partially purified bone-inducing protein preparations. Bone formation is said to require the presence of at least 5%, and preferably at least 10%, non-fibrillar collagen. The authors claim that removal of telopeptides which are responsible in part for the immunogenicity of collagen preparations is more suitable for xenogenic implants.

European Patent Application Serial No. 309,241, published 3/29/89, discloses a device for inducing endochondral bone formation comprising an osteogenic protein preparation, and a matrix carrier comprising 60-98% of either mineral component or bone collagen powder and 2-40% atelopeptide hypoimmunogenic collagen.

Deatherage et al. (1987) Collagen Rel. Res. 7:2225-2231, purport to disclose an apparently xenogenic implantable device comprising a bovine bone matrix extract that has been minimally purified by a one-step ion exchange column and reconstituted, highly purified human Type-I placental collagen.

U.S. 3,394,370, published 7/19/83, describes a matrix of reconstituted collagen purportedly useful in xenogenic implants. The collagen fibers are treated enzymatically to remove potentially immunogenic telopeptides (also the primary source of interfibril crosslinks) and are dissolved to remove associated non-collagen components. The matrix is formulated by dispersing the reconstituted collagen in acetic acid to form a disordered matrix of elementary collagen molecules that is then mixed with osteogenic factor and lyophilized to form a "semi-rigid foam or sponge" that is preferably crosslinked. The formulated matrix is not tested in vivo.

U.S. 4,172,128, published 10/23/79, describes a method for degrading and regenerating bone-like material of reduced immunogenicity, said to be useful cross-species. Demineralized bone particles are treated with a swelling agent to dissolve any associated mucopolysaccharides (glycosaminoglycans) and the collagen fibers subsequently dissolved to form a homogenous colloidal solution. A gel of reconstituted fibers then can be formed using physiologically inert mucopolysaccharides and an electrolyte to aid in fibril formation.

Described herein are osteogenic devices comprising matrices containing dispersed osteogenic protein produced from recombinant DNA and capable of bone induction in allogenic and xenogenic implants. Also described are recombinant osteogenic proteins expressed from mammalian cells and capable of inducing endochondral bone formation in mammals, including humans. Also described are genes encoding osteogenic proteins and methods for their production using recombinant DNA techniques. Also described is a biocompatible, in vivo biodegradable matrix capable, in combination with an osteoinductive protein, of producing endochondral bone formation in mammals, including humans.

These and other objects and features of the invention will be apparent from the description, drawings, and claims which follow.

### Summary of the Invention

Described herein are osteogenic proteins and devices which, when implanted in a mammalian body, can induce at the locus of the implant the full developmental cascade of endochondral bone formation including vascularization, mineralization, and bone marrow differentiation. The devices comprise a carrier material, referred to herein as a matrix, having the characteristics disclosed below, and containing dispersed osteogenic protein produced using recombinant DNA techniques and expressed from eukaryotic cells, preferably mammalian cells.

Preferred embodiments of the recombinant protein dispersed in the matrix disclosed herein closely mimic the physiological activity of native form protein extracted from natural sources and reconstituted in allogenic demineralized bone powder matrix material. The preferred proteins have a specific activity far higher than any biosynthetic material heretofore reported, an activity which, within the limits of precision of the activity assay, appears essentially identical to the substantially pure material produced as set forth in copending application Serial No. 179,406 filed April 8, 1988 (PCT US/89 01453). Thus, this application discloses how to make and use osteogenic devices which induce the full developmental cascade of endochondral bone formation essentially as it occurs in natural bone healing.

A key to these developments was the elucidation of amino acid sequence and structure data of native osteogenic protein. A protocol was developed which results in retrieval of active, substantially pure osteogenic protein from mammalian bone having a half-maximum bone forming activity of about 0.8 to 1.0 ng per mg of implant. The availability of the material enabled the inventors to elucidate all structural details of the protein necessary to achieve bone formation. Knowledge of the protein's amino acid sequence and other structural features enabled the identification and cloning of native genes.

Consensus DNA sequences based on partial sequence data and observed homologies with regulatory proteins disclosed in the literature were used as probes for extracting genes encoding osteogenic protein from genomic and cDNA libraries. One of the consensus sequence probes isolated a previously unidentified DNA sequence, portions of which, when ligated, encoded a protein comprising a region capable of inducing endochondral bone formation when properly modified, incorporated in a suitable matrix, and implanted as disclosed herein. The protein, referred to herein as OP1, as well as various truncated forms and fusion constructs, has been expressed in E. coli and various mammalian cells from the full length cDNA sequence and various truncated synthetic DNAs, and has been discovered to exhibit osteogenic activity as a homodimer or as a heterodimer with BMP2, another osteogenic protein extracted from human DNA libraries with the consensus sequence probes.

Characterization of the OP1 gene and identification of the DNA and amino acid sequence necessary for activity has allowed expression of the gene in mammalian cells. Mammalian cell expression of recombinant proteins, particularly mammalian proteins intended for therapeutic use, is generally thought to yield proteins having a structure most like that of the natural material. This is particularly true for secreted proteins which require particular post-translational modifications, such as glycosylation, which are not carried out in procaryotic systems. While expression of the OP1 gene in E. coli has shown that the unglycosylated form of the protein has osteogenic activity, there may be other as yet undetermined functions for the oligosaccharides, relating to protein stability, solubility, or immunogenicity, for example. In addition, purification of proteins secreted into culture media provide an alternative to extraction of induced proteins from procaryotic inclusion bodies.

Mammalian cell expression of the gene also has allowed determination of the N-terminus of the mature protein. The amino acid sequence of what is believed to be the mature form of OP1 is (Seq. ID No. 1):

Recombinantly-produced OP1 also is active in several forms truncated at the protein's N-terminus. One main species of truncated OP1 is (Seq. ID No. 2):

Four other active shorter OP1 sequences are:

These 6 species of OP1 have been tested for osteogenic activity in vivo and all have been shown to induce endochondral bone formation in a dose-dependent manner when implanted in a mammal in association with a suitable matrix. The specific activity of these species is close to that of the substantially pure, naturally-sourced osteogenic protein. Moreover, these proteins mimic the activity of the naturally-sourced material more closely than other osteogenic protein preparations heretofore reported.

Recombinantly produced OP1 is expressed as a glycosylated homodimer in mammalian cells. Homodimers of OP1-18 have an apparent molecular weight of about 36 kD, when oxidized, and about 18 kD when reduced, as determined by SDS-PAGE gels. OP1-16S, OP1-16V, OP1-16M, OP1-16A and OP1-16L have an apparent molecular weight of about 16kD, when reduced, and homodimers of these proteins, as well as heterodimers with OP1-18 have an apparent molecular weight within the range of about 30-36 kD when oxidized, as determined by SDS-PAGE gels. In the reduced state, these proteins have no detectable osteogenic activity.

OP1 now has been expressed in a number of different mammalian cells, all of which glycosylate and process the protein after translation. While the precise structure of the oligosaccharide side chains may vary among the different cell lines, in all cases the expressed sequence is osteogenically active in a specific and dose dependent manner.

The invention is not limited to those specific constructs. Thus, the osteogenic proteins of this invention may include forms having varying glycosylation patterns, varying N-termini, a family of related proteins having regions of amino acid sequence homology, and active truncated or mutated forms of the native amino acid sequence, produced by expression of recombinant DNA in eucaryotic host cells. Active sequences useful in an osteogenic device of this invention is envisioned to include osteogenic proteins having at least a 70% sequence homology, preferably at least 80%, with the amino acid sequence of OP1-16V. This includes longer forms of the protein, as well as allelic variants and muteins.

Thus, in view of this disclosure, skilled genetic engineers can isolate genes from cDNA or genomic libraries which encode appropriate amino acid sequences, or construct DNAs from oligonucleotides, and then can express them in various types of eucaryotic host cells to produce large quantities of active proteins capable of inducing bone formation in mammals, including humans.

The osteogenic proteins are useful in clinical applications in conjunction with a suitable delivery or support system (matrix). The matrix comprises biocompatible, protein-extracted, mineral-free, delipidated, insoluble Type-I bone collagen particles which may be allogenic or xenogenic to the host. The particles preferably are treated with a fibril-modifying agent such as hot water or other fibril-modifying solvents, to alter the particle morphology, i.e., to increase the intraparticle porosity and the surface area of the particles. The particles are packed together to form the matrix. The spaces among the particles must be of a dimension to permit progenitor cell migration and subsequent cell differentiation and proliferation. The particle size should be within the range of 70 - 850 µm, preferably 150µm - 420µm. The matrix may be fabricated by close packing the particles into a shape spanning the bone defect, or by otherwise shaping the packed particles as desired. The matrix is biocompatible (non-inflammatory) and biodegradable in vivo, and serves as a "temporary scaffold" and substratum for recruitment of migratory progenitor cells, and as a base for their subsequent anchoring and proliferation. As disclosed herein, the matrix may be combined with osteogenic protein to induce endochondral bone formation reliably and reproducibly in a mammalian body.

The development of this matrix material resulted from the discovery of key features required for successful implantation of xenogenic bone matrix and osteogenic protein. Studies indicated that osteogenic devices comprising substantially pure osteogenic protein and allogenic demineralized, delipidated protein-extracted bone matrices must have interstices dimensioned to permit the influx, proliferation and differentiation of migratory progenitor cells. It was also observed that osteogenic devices comprising xenogenic bone matrices induce little or no endochondral bone formation in vivo. The absence of bone formation by xenogenic matrices generally has been thought to be due to an immunogenic or inhibitory response to components still present in the matrix (e.g., the collagen telopeptides or associated non-collagenous glycoproteins.)

It has now been discovered that the overall specific particle surface area (surface area/unit mass), the degree of porosity and micropitting, and the size of the micropits and pores of the matrix particles is important for successful xenogenic implants, and even for allogenic implants of certain species.

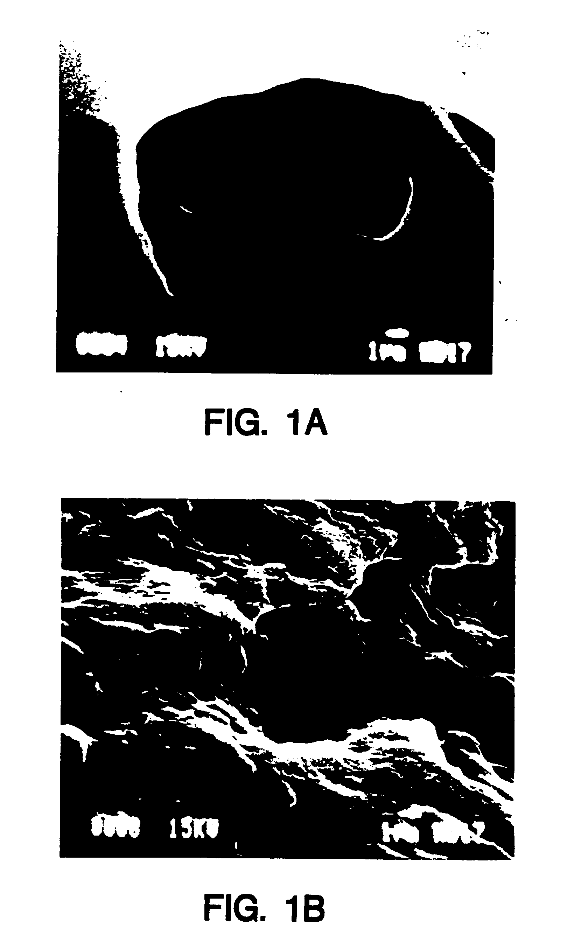

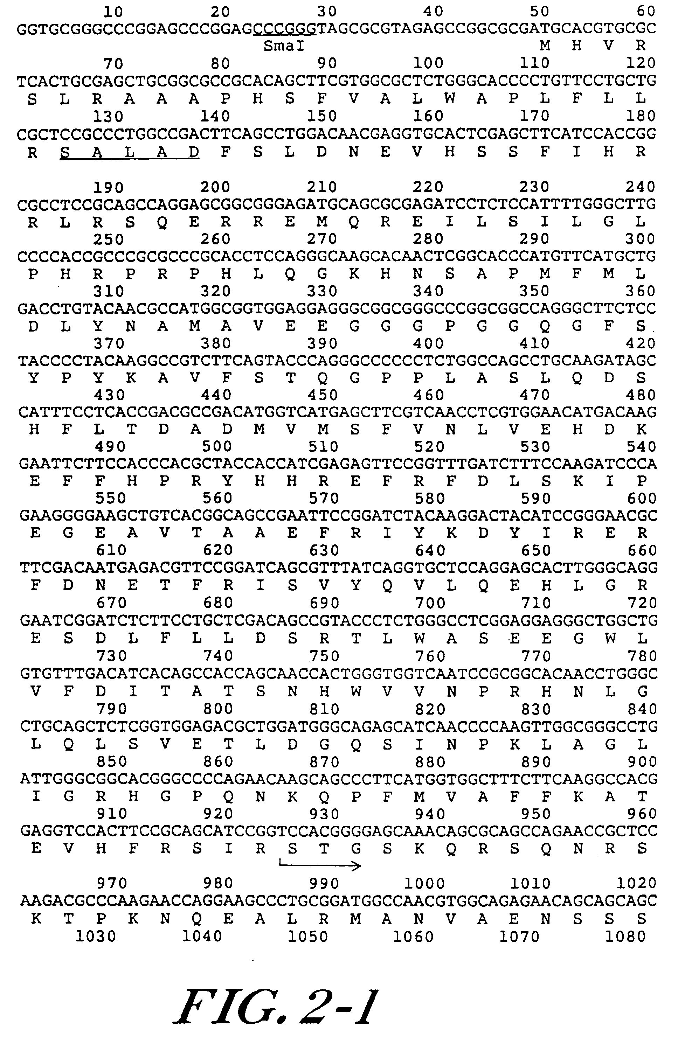

Panels A and B of FIGURE 1 are scanning electron micrographs showing the particle structure of demineralized, guanidine-extracted bone matrix from rat and calf, respectively. As can be seen from the SEMs, there is a significantly greater inherent porosity, or surface area, in rat bone matrix than in bovine bone matrix. It has been discovered that increasing the porosity and intraparticle surface area of bone matrix can promote osteogenic induction as evidenced by rat collagenous bone matrix implants. This is achieved by treating collagenous bone matrix with certain solvents or heat so as to alter its morphology. Agents suitable for this purpose are disclosed herein and are termed collagen fibril-modifying agents.

Thus, osteogenic devices may comprise matrices which have been treated to increase the surface area and porosity of matrix collagen particles substantially.

The currently preferred fibril-modifying agent useful in the osteogenic devices of this invention is a heated aqueous medium, most preferably water. Heating demineralized delipidated guanidine extracted bone collagen in water at high temperature (37°-65°, preferably 45°-60°C) for approximately one hour is generally sufficient to achieve the desired surface morphology. Although the mechanism is not clear, it is hypothesized that the heat treatment alters the collagen fibrils, resulting in an increase in the particle's surface area. Thus, bone matrix may be treated at various elevated temperatures in water (lg/30ml) with stirring and then filtered. Treatment of insoluble collagen in water by increasing temperature results initially in a melting transition (Tm), the temperature required to go from one-quarter to three quarters of the total transition from helical structure to non-helical. Thereafter the fibrils will shrink abruptly a fraction of length at some higher temperature, designated as the shrinkage temperature (Ts). Ts is normally higher than Tm, reflecting the added stability contributed by molecular packing. At pHs below approximately 5, both the Tm and Ts values decrease for heated collagen.

Examination of solvent treated bone collagenous matrix shows that demineralized guanidine-extracted xenogenic bovine bone comprises a mixture of additional materials and that extracting these materials can improve matrix properties. Chromatographic separation of components in the extract, followed by addition back to active matrix of the various extract fractions corresponding to the chromatogram peaks, indicates that there is a fraction which can inhibit the osteoinductive effect. The identity of the substance or substances in this inhibiting fraction has not as yet been determined. In one aspect of this invention, a matrix is provided comprising Type-I bone collagen particles of the type described above, further characterized in that they are depleted in this inhibiting component.

In view of this disclosure, one skilled in the art can create a biocompatible matrix of choice having a desired porosity or surface microtexture useful in the production of osteogenic devices, and useful in other implantable contexts, e.g., as a packing to promote bone induction, or as a biodegradable sustained release implant.

The osteogenic proteins and implantable osteogenic devices disclosed herein will permit the physician to obtain optimal predictable bone formation to correct, for example, acquired and congenital craniofacial and other skeletal or dental anomalies (Glowacki et al. (1981) Lancet 1:959-963). The devices may be used to induce local endochondral bone formation in non-union fractures as demonstrated in animal tests, and in other clinical applications including periodontal applications where bone formation is required. Another potential clinical application is in cartilage repair, for example, in the treatment of osteoarthritis.

### Brief Description of the Drawing

The foregoing and other objects of the invention, the various features thereof, as well as the invention itself, may be more fully understood from the following description, when read together with the accompanying drawings, in which:
FIGURE 1A and 1B are scanning electron micrographs (5000X) of demineralized, delipidated (A) rat bone collagen particles, and (B) bovine bone collagen particles;
FIGURE 2-1 and 2-2 represent the full length cDNA and encoded amino acid sequence of the prepro form of human OP1 protein (Seq. ID No. 7);
FIGURE 3A through 3F are restriction maps of various expression vectors designed for the mammalian cell expression of OP1;
FIGURE 4 is a photoreproduction of western blots (immunoblots) comparing OP1 expressed from: COS cells - (A) pH717, (B) pH731; CHO cells - (C) pH754, (D) pH752; and BSC cells - (E) pH717, (F) pW24;
FIGURE 5A-C are (1) elution profiles and (2) photoreproductions of SDS-PAGE gels expressed from BSC cells and purified (in order) on: (A) S-Sepharose, (B) phenyl-Sepharose, and (c) C-18 columns;
FIGURE 6 is a photoreproduction of SDS-PAGE gels of OP1 purified from BSC cells, comparing the intact dimer under oxidized conditions (36 kD, lane 1) and the corresponding monomer, after reduction with dithiothreitol (18kD, lane 5), with molecular weight standards (lanes 2-4);

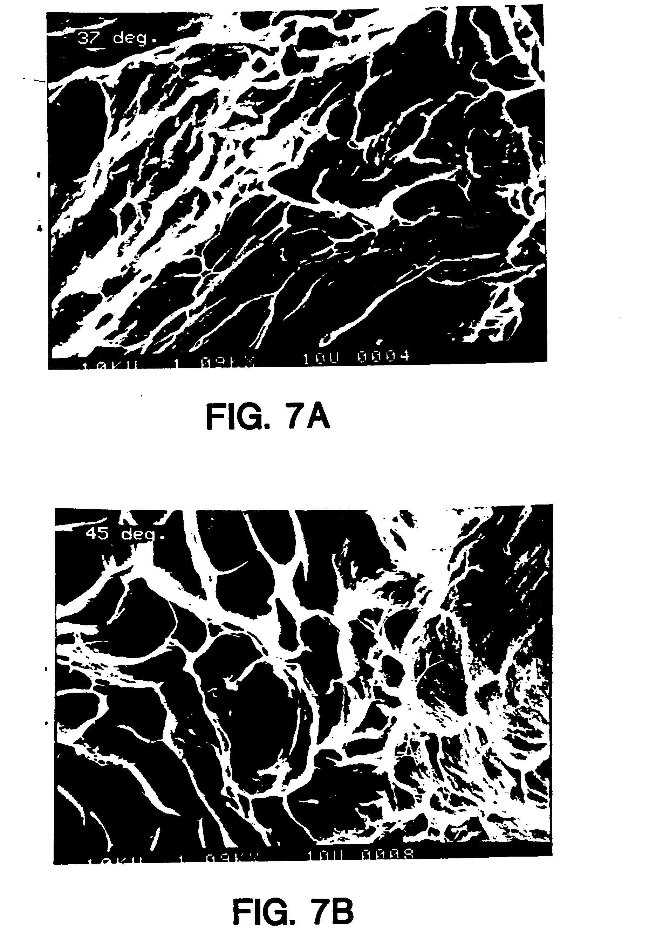

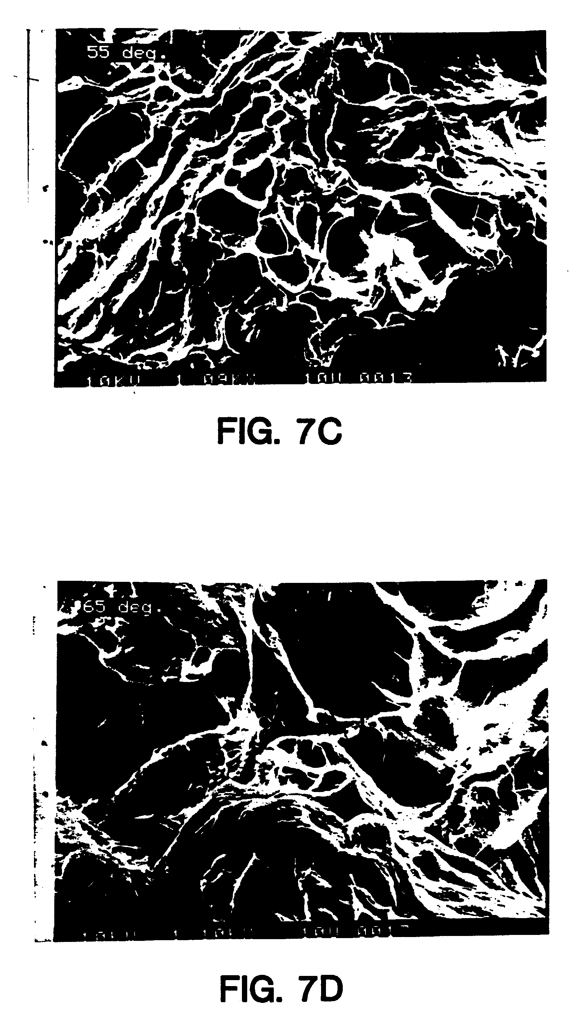

FIGURE 7A through 7D are scanning electron micrographs (approx. 1000X) of demineralized, delipidated bovine bone matrix heat treated in water at (A) 37° C, (B) 45° C, (C) 55° C, and (D) 65° C;
FIGURE 8 is a 214 nm absorbance tracing of the extract isolated from hot water-treated bovine matrix, identifying the inhibitory effect of individual fractions on in vivo bone formation;
FIGURE 9A and 9B are bar graphs showing the inhibitory effect of hot water-treated matrix extract on OP1 activity, as measured by (A) alkaline phosphatase activity and (B) calcium content in day 12 implants, vs. increasing concentration of extract solvent;
FIGURE 10A-F are photomicrographs (220x) of allogenic implants of OP1 expressed from COS, BSC and CHO cells, and which follow the developmental cascade of endochondral bone osteogenesis;
FIGURE 11 is a photomicrograph showing the histology (day 12) of a xenogenic implant of this invention using OP1 expressed from BSC cells and hot water-treated xenogenic bovine matrix;
FIGURE 12 describes the dose dependence of osteogenic implants for day 12 implants, as determined by alkaline phosphatase activity and calcium content, for allogenic implants containing OP1 expressed from COS, BSC and CHO cells; and
FIGURE 13A and 13B are bar graphs showing the dose dependence of OP1 expressed in COS and BSC cells, as measured by (A) alkaline phosphatase activity and (B) calcium content in xenogenic implants (day 12), vs increasing concentration of protein (dose curve in ng).

### Description

Purification protocols first were developed which enabled isolation of the osteogenic protein present in crude protein extracts from mammalian bone. (See PCT US 89/01453, and U.S. Serial No. 179,406 filed April 8, 1988). The development of the procedure, coupled with the availability of fresh calf bone, enabled isolation of substantially pure bovine osteogenic protein (BOP). BOP was characterized significantly; its ability to induce cartilage and ultimately endochondral bone growth in cat, rabbit, and rat were demonstrated and studied; it was shown to be able to induce the full developmental cascade of bone formation previously ascribed to unknown protein or proteins in heterogeneous bone extracts. This dose dependent and highly specific activity was present whether or not the protein was glycosylated (see U.S. Serial No. 232,630 filed 8/15/88 and Sampath et al., (1990) J. Biol. Chem. 265: pp. 13198-13205). Sequence data obtained from the bovine materials suggested probe designs which were used to isolate human genes. The OP human counterpart proteins have now been expressed and extensively characterized.

These discoveries enabled preparation of DNAs encoding totally novel, non-native protein constructs which individually as homodimers and combined with other species as heterodimers are capable of producing true endochondral bone (see PCT 89/01469, filed 4/7/89 and US Serial No. 315,342, filed 2/23/89). They also permitted expression of the natural material, truncated forms, muteins, analogs, fusion proteins, and various other variants and constructs, from cDNAs and genomic DNAs retrieved from natural sources or from synthetic DNA produced using the techniques disclosed herein and using automated, commercially available equipment. The DNAs may be expressed using well established molecular biology and recombinant DNA techniques in procaryotic or eucaryotic host cells, and may be oxidized and refolded in vitro if necessary, to produce biologically active protein.

One of the DNA sequences isolated from genomic and cDNA libraries encoded a previously unidentified gene, referred to herein as OP1. The protein encoded by the isolated DNA was identified originally by amino acid homology with proteins in the TGF-β family. Consensus splice signals were found where amino acid homologies ended, designating exon-intron boundaries. Three exons were combined to obtain a functional TGF-β-like domain containing seven cysteines. (See, for example, U.S. Serial No. 315,342 filed 2/23/80, or Ozkaynak, E. et al., (1990) EMBO. 9: pp. 2085-2093).

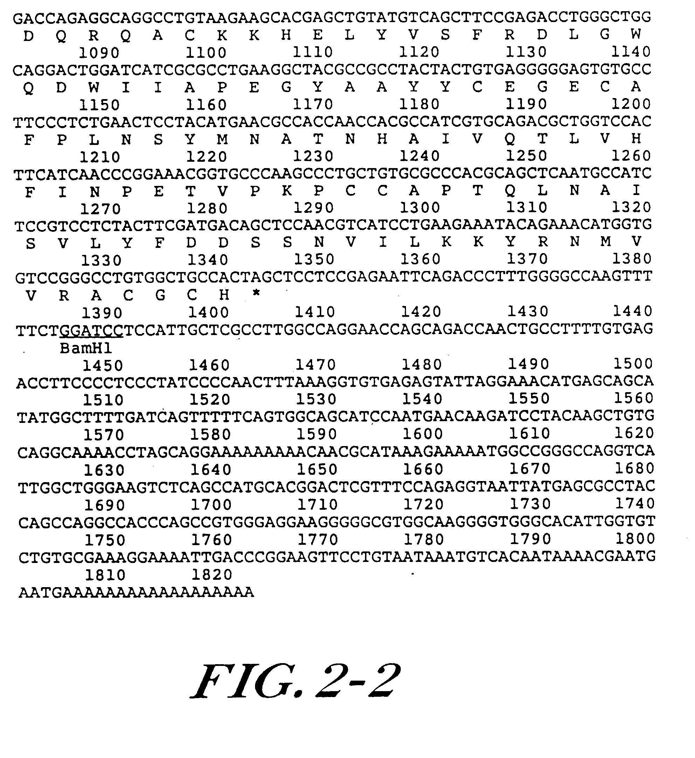

The full-length cDNA sequence for OP1, including the amino acid sequence it encodes, is represented in Figure 2. This full length cDNA sequence of OP1, as well as various truncated forms of the gene, and fused genes, have been expressed in E. coli and shown to have osteogenic activity when implanted in a mammal in association with a matrix.

The native form protein is expressed originally in a "prepro" form which includes a signal peptide sequence for appropriate secretion of the protein. The signal peptide cleavage site is underlined in Figure 2. Removal of the signal peptide yields the "pro" form of the protein, which is processed upon secretion to yield the mature sequence. The cleavage site yielding the mature sequence is indicated by an arrow in Figure 2. The amino acid sequence of what is believed to be the mature form is (Seq. ID No. 1):

Both the pro form and prepro form, when properly dimerized, folded, adsorbed on a matrix, and implanted, display osteogenic activity, presumably due to proteolytic degradation resulting in cleavage and generation of mature form protein or active truncated analogs.

Active OP1 can also be purified in a truncated form, missing part of the protein's N terminus. One active truncated form of OP1 is (Seq. ID No. 2):

Four other active truncated forms of OP1 are:

Given the foregoing amino acid and DNA sequence information, various DNAs can be constructed which encode at least the minimal active domain of OP1, and various analogs thereof, as well as fusion proteins, other truncated forms of the mature proteins, and similar constructs. These DNAs can be produced by those skilled in the art using well known DNA manipulation techniques involving genomic and cDNA isolation, construction of synthetic DNA from synthesized oligonucleotides, and cassette mutagenesis techniques. 15-100mer oligonucleotides may be synthesized on a Biosearch DNA Model 8600 Synthesizer, and purified by polyacrylamide gel electrophoresis (PAGE) in Tris-Borate-EDTA buffer. The DNA may then be electroeluted from the gel. Overlapping oligomers may be phosphorylated by T4 polynucleotide kinase and ligated into larger blocks which may also be purified by PAGE.

The cDNA or synthetic DNA then may be integrated into an expression vector and transfected into an appropriate host cell for protein expression. The host may be a procaryotic or eucaryotic cell since the former's inability to glycosylate protein will not destroy the protein's osteogenic activity. Useful host cells include E. coli, Saccharomyces, the insect/baculovirus cell system, myeloma cells, and various mammalian cells. The protein of this invention preferably is expressed in mammalian cells, as disclosed herein. The vector additionally may encode various sequences to promote correct expression of the recombinant protein, including transcription promoter and termination sequences, enhancer sequences, preferred ribosome binding site sequences, preferred mRNA leader sequences, preferred signal sequences for protein secretion, and the like. The DNA sequence encoding the gene of interest also may be manipulated to remove potentially inhibiting sequences or to minimize unwanted secondary structure formation. The recombinant osteogenic protein also may be expressed as a fusion protein. After being translated, the protein may be purified from the cells themselves or recovered from the culture medium. All biologically active protein forms comprise dimeric species joined by disulfide bonds or otherwise associated, produced by oxidizing and refolding one or more of the various recombinant proteins within an appropriate eucaryotic cell or in vitro after expression of individual subunits.

As stated earlier, it is generally held that recombinant production of mammalian proteins for therapeutic uses are preferably expressed in mammalian cell culture systems in order to produce a protein whose structure is most like that of the natural material. Recombinant protein production in mammalian cells requires the establishment of appropriate cells and cell lines that are easy to transfect, are capable of stably maintaining foreign DNA with an unrearranged sequence, and which have the necessary cellular components for efficient transcription, translation, post-translation modification, and secretion of the protein. In addition, a suitable vector carrying the gene of interest also is necessary. DNA vector design for transfection into mammalian cells should include appropriate sequences to promote expression of the gene of interest as described supra, including appropriate transcription initiation, termination, and enhancer sequences, as well as sequences that enhance translation efficiency, such as the Kosak consensus sequence. Preferred DNA vectors also include a marker gene and means of amplifying the copy number of the gene of interest.

Substantial progress in the development of mammalian cell expression systems has been made in the last decade and many aspects of the system are well characterized. A detailed review of the state of the art of the production of foreign proteins in mammalian cells, including useful cells, protein expression-promoting sequences, marker genes, and gene amplification methods, is disclosed in Bendig, Mary M., (1988) Genetic Engineering, 7:91-127.

Briefly, among the best characterized transcription promoters useful for expressing a foreign gene in a particular mammalian cell are the SV40 early promoter, the adenovirus promoter (AdMLP), the mouse metallothionein-I promoter (mMT-I), the Rous sarcoma virus (RSV) long terminal repeat (LTR), the mouse mammary tumor virus long terminal repeat (MMTV-LTR), and the human cytomegalovirus major intermediate-early promoter (hCMV). The DNA sequences for all of these promoters are known in the art and are available commercially.

One of the better characterized methods of gene amplification in mammalian cell systems is the use of the inducible DHFR gene in a dhfr- cell line. Generally, the DHFR gene is provided on the vector carrying the gene of interest, and induction by addition of the cytotoxic drug methotrexate amplifies the DHFR gene copy number, as well as that of the associated gene of interest. DHFR as an inducible, amplifying marker gene in transfected chinese hamster ovary cell lines (CHO cells) is particularly well characterized in the art. Other genes useful as inducible gene amplifiers include the adenosine deaminase (ADA) and glutamine synthetase (GS) genes.

The choice of cells/cell lines is also important and depends on the needs of the experimenter. Monkey kidney cells (COS) provide high levels of transient gene expression, providing a useful means for rapidly testing vector construction and the expression of cloned genes. COS cells are transfected with a simian virus 40 (SV40) vector carrying the gene of interest. The transfected COS cells eventually die, thus preventing the long term production of the desired protein product. However, transient expression does not require the time consuming process (often several weeks) required for the development of a stable cell line.

Among established cell lines, CHO cells may be the best characterized to date. They also are capable of expressing proteins from a broad range of cell types. The general applicability of CHO cells and its successful production for a wide variety of human proteins in unrelated cell types emphasizes the underlying similarity of all mammalian cells. Thus, while the glycosylation pattern on a recombinant protein produced in a mammalian cell expression system may not be identical to the natural protein, the differences in oligosaccharide side chains are often not essential for biological activity of the expressed protein.

Methods for expressing and purifying recombinant OP1 from a variety of mammalian cells, the nature of the xenogenic matrix, and other material aspects concerning the nature, utility, and how to make and how to use the subject matter claimed will be further understood from the following, which constitutes the best method currently known for practicing the invention.

### I. RECOMBINANT PROTEIN EXPRESSION IN MAMMALIAN CELLS

Several different mammalian cell expression systems have been used to express recombinant OP1 proteins of this invention. In particular, COS cells are used for the rapid assessment of vector construction and gene expression, using an SV40 vector to transfect the DNA sequence into COS cells. Stable cell lines are developed using CHO cells (chinese hamster ovary cells) and a temperature-sensitive strain of BSC cells (simian kidney cells, BSC40-tsA58, (1988) Biotechnology 6: 1197-1196) for the long term production of OP1. Two different promoters are used to transcribe OP1: the CMV promoter, boosted by the enhancer sequence from the Rous sarcoma virus LTR, and the mMT promoter (mouse metallothionein promoter). Several selection marker genes also are used, namely, neo (neomycin) and DHFR. The DHFR gene also may be used as part of a gene amplification scheme for CHO cells. Another gene amplification scheme relies on the temperature sensitivity (ts) of BSC40-tsA58 cells transfected with an SV40 vector. Temperature reduction to 33°C stabilizes the ts SV40 T antigen which leads to the excision and amplification of the integrated transfected vector DNA, thereby also amplifying the associated gene of interest.

Stable cell lines were established for CHO cells as well as BSC40-tsA58 cells (hereinafter referred to as "BSC cells"). The various cells, cell lines and DNA sequences chosen for mammalian cell expression of the OP1 proteins of this invention are well characterized in the art and are readily available. Other promoters, selectable markers, gene amplification methods and cells also may be used to express the OP1 proteins of this invention, as well as other osteogenic proteins. Particular details of the transfection, expression, and purification of recombinant proteins are well documented in the art and are understood by those having ordinary skill in the art. Further details on the various technical aspects of each of the steps used in recombinant production of foreign genes in mammalian cell expression systems can be found in a number of texts and laboratory manuals in the art, such as, for example, Current Protocols in Molecular Biology, F.M. Ausubel et al., ed., John Wiley & Sons, New York 1987.

### 1. Exemplary Expression Vectors

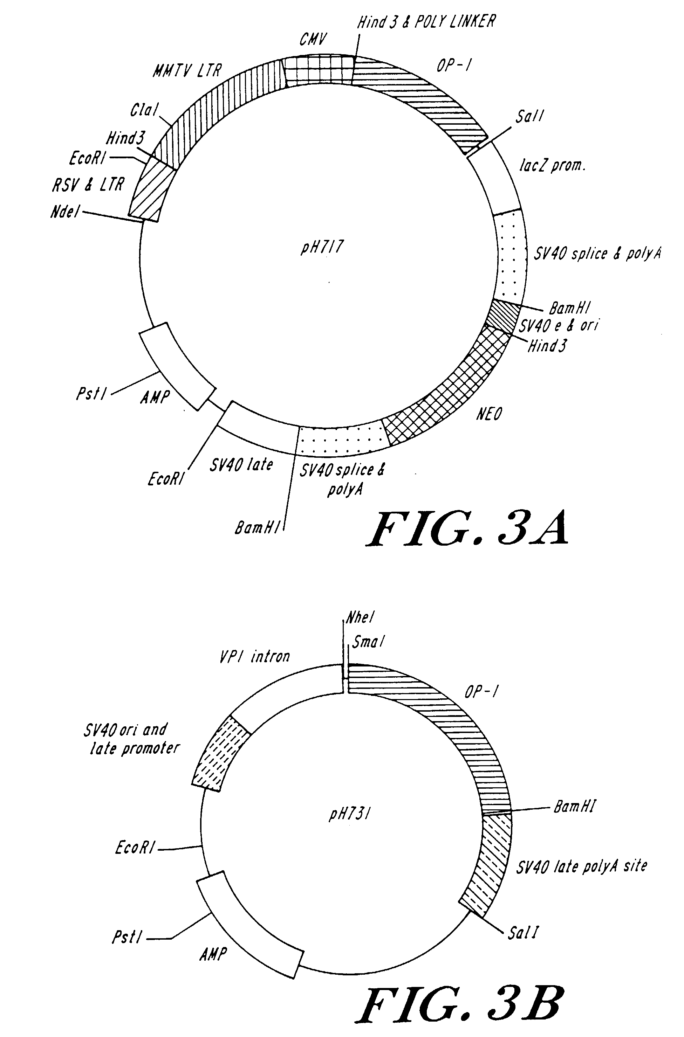

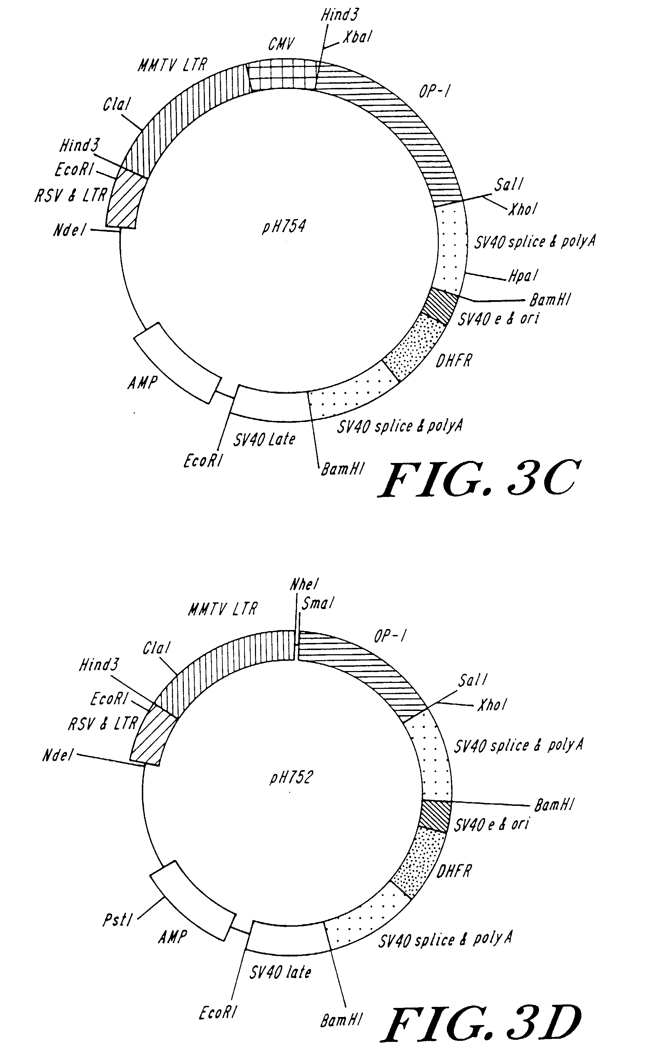

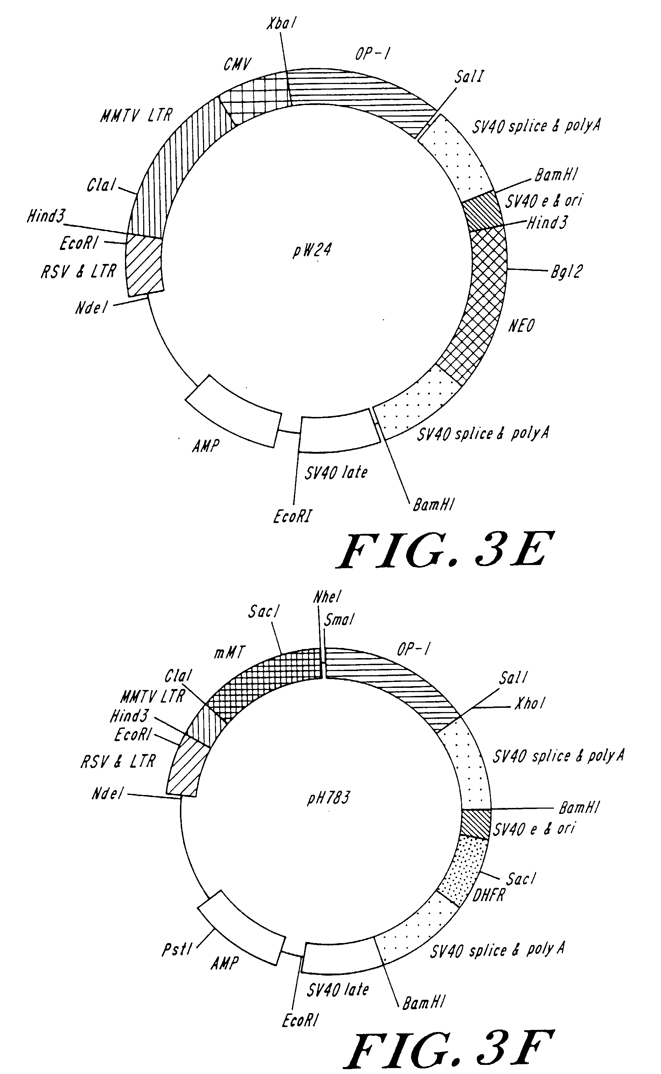

Figure 3 discloses restriction maps of various exemplary expression vectors designed for OP1 expression in mammalian cells. Each of these vector contructs employs a full-length cDNA sequence originally isolated from a human cDNA library (human placenta) and subsequently cloned into a conventional pUC vector (pUC-18) using pUC polylinker sequences at the insertion sites. The OP1 cDNA fragment cloned into each of these constructs is either the intact SmaI-BamHI OP1 cDNA fragment depicted in Figure 2 (Seq. ID No.7), or modifications of this fragment where the flanking non-coding 5' and/or 3' sequences have been trimmed back, using standard molecular biology methodology. Each vector also employs an SV40 origin of replication (ori), useful for mediating plasmid replication in primate cells (e.g., COS and BSC cells). In addition, the early SV40 promoter is used to drive transcription of marker genes on the vector (e.g., neo and DHFR).

The pH717 expression vector (Fig. 3A) contains the neomycin (neo) gene as an inducible selection marker. This marker gene is well characterized in the art and is available commercially. Alternatively, other selectable markers may be used. The particular vector used to provide the neo gene DNA fragment for pH717 may be obtained from Clontech, Inc., Palo Alto, CA (pMAM-neo-blue). In pH717, OP1 DNA transcription is driven by the CMV promoter, boosted by the RSV-LTR and MMTV-LTR (mouse mammary tumor virus) enhancer sequences. These sequences are known in the art, and are available commercially. For example, vectors containing this promoter/enhancer sequence may be obtained from Invitrogen Inc., San Diego, CA, (e.g., pCDM8).

Expression vector pH731 (Fig.3B), utilizes the SV40 late promoter to drive OP1 transcription. As indicated above, the sequence and characteristics of this promoter also are well known in the art. Alternatively, pH731 may be generated by inserting the SmaI-BamHI fragment of OP1 into pEUK-Cl (Clontech, Inc., Palo Alto, CA).

The pH754 expression vector (Fig. 3C) contains the DHFR sequence as both a selection marker and as an inducible gene amplifier. OP1 is under CMV control. The DNA sequence for DHFR and is well characterized in the art, and is available commercially. Alternatively, pH754 may be generated from pMAM-neo (Clontech, Inc., Palo Alto, CA) by replacing the neo gene (BamHI digest) with a BamHI fragment containing the DHFR gene (e.g., obtained from pSV5-dhfr (ATCC #37148)). OP1 DNA then can be inserted into the polylinker site downstream of the MMTVLTR sequence (mouse mammary tumor virus LTR), yielding pH752 (Fig. 3D). The CMV promoter sequence then may be inserted into pH752 (opened at Clal-Nhel) as a Clal-Xbal fragment (e.g., from pMAM-neo blue, Clontech, Inc.).

The pW24 vector (Fig. 3E), is essentially identical in sequence to p754, except that neo is used as the marker gene (see pH717), in place of DHFR.

Similarly, pH783 (Fig. 3F) contains the amplifiable marker DHFR, but here OP1 is under mMT (mouse metallothionein promoter) control. The mMT promoter is well characterized in the art and is available commercially. Alternatively, a Clal-Nhel fragment containing the mMT promoter sequence (available from Allegro Nichols Institute Diagnostics, San Juan Capistrano, CA) can be inserted into pH752 to generate pH783.

All vectors tested are stable in the various cells used to express OP1, and provide a range of OP1 expression levels.

### 2. Exemplary Mammalian Cells

Recombinant OP1 has been expressed in three different cell expression systems: COS cells for rapidly screening the functionality of the various expression vector constructs, CHO cells for the establishment of stable cell lines, and BSC40-tsA58 cells as an alternative means of producing OP1 protein.

### A. COS CELLS

COS cells (simian kidney cells) are used for rapid screening of vector contructs and for immediate, small scale production of OP1 protein. COS cells are well known in the art and are available commercially. The particular cell line described herein may be obtained through the American Type Culture Collection (ATCC #COS-1, CRL-1650).

OP1 expression levels from different vectors, analyzed by northern and western blot assays, are compared in Table I below:

**TABLE 1**

| ANALYSIS OF OP1 EXPRESSION IN COS CELLS | | |
|---|---|---|
| Vector | mRNA | OP1 Production |
| pH717 | +++ | ++ |
| pH731 | + | + |
| pH752 | +++ | ++++ |
| pH754 | +++ | ++++ |

pH754-transfected COS cells appear to produce the highest yield of OP1 to date. However, because transfected COS cells do not divide and die several days post-transfection, large amounts of plasmid DNA are required for each scaled up transformation.

Large scale preparations of OP1 from transfected COS cells may be produced using conventional roller bottle technology. Briefly, 14 X 10⁶ cells are used to seed each bottle. After 24 hrs of growth, the cells are transfected with 10 µg of vector DNA (e.g., pH717) per 10⁶ cells, using the DEAE-dextran method. Cells are then conditioned in serum-free media for 120 hr before harvesting the media for protein analysis. Following this protocol, OP1 yield is approximately 2-6 ng/ml.

### B. CHO Cells

CHO cells (chinese hamster ovary cells) may be used for long term OP1 production. CHO cell lines are well characterized for the small and large scale production of foreign genes and are available commercially. The particular cell line described herein is CHO-DXB11, (Laurence Chasin, Columbia University, NY). Table II, below, shows exemplary OP1 yields obtained with a variety of expression vectors.

**TABLE II**

| CHO Cells | Plasmid | Selection Marker | OP1 Production ng/ml |
|---|---|---|---|
| | pH717 | NEO | 2-5 |
| * | pH752/pH754 | DHFR | 100-150 |

| | | | |
|---|---|---|---|
| *Cells are adapted to grow in 0.1 uM methotrexate | | | |

CHO cells may be transfected by conventional calcium phosphate technique. CHO cells preferably are transfected with pH754 or pH752 and are conditioned in media containing serum proteins, as this appears to enhance OP1 yields. Useful media include media containing 0.1-0.5% dialyzed fetal calf serum (FCS).

### C. BSC CELLS

The BSC40-tsA58 cell line ("BSC cells") is a temperature-sensitive strain of simian kidney cells (1988, Biotechnology 6: 1192-1196) which overcomes some of the problems associated with COS cells. These BSC cells have the advantage of being able to amplify gene sequences rapidly on a large scale with temperature downshift, without requiring the addition of exogenous, potentially toxic drugs. In addition, the cells may be recycled. That is-, after induction and stimulation of OP1 expression, the cells may be transferred to new growth medium, grown to confluence at 39.5°C and induced a second time by downshifting the temperature to 33°C. BSC cells may be used to establish stable cell lines rapidly for protein production.

Transfected BSC cells may be induced by shifting the temperature down to 33°C, in media containing 10% FCS, and harvesting the conditioned media after 96 hrs of incubation. Comparable amounts of OP1 RNA and protein are obtained, as compared with CHO cells (e.g., 100-150 ng OP1/ml conditioned media from BSC clones transfected with pH717).

### 3. Evaluation of OP1 transfected cells

Expression levels of transfected OP1 sequences can be measured in the different systems by analyzing mRNA levels (Northern blots), using total cellular RNA and conventional hybridization methodology. Generally, about 1 X 10⁶ cells are needed for mRNA analysis. Data between individual cell lines can be compared if the total number of cells and the total amount of mRNA is normalized, using rRNA as an internal standard. Ribosomal RNA is visualized in the agarose gel by ethydium bromide stain prior to transfer of the RNA to nitrocellulose sheets for hybridization. Ribosomal RNA also provides an indicator of the integrity of the RNA preparation.

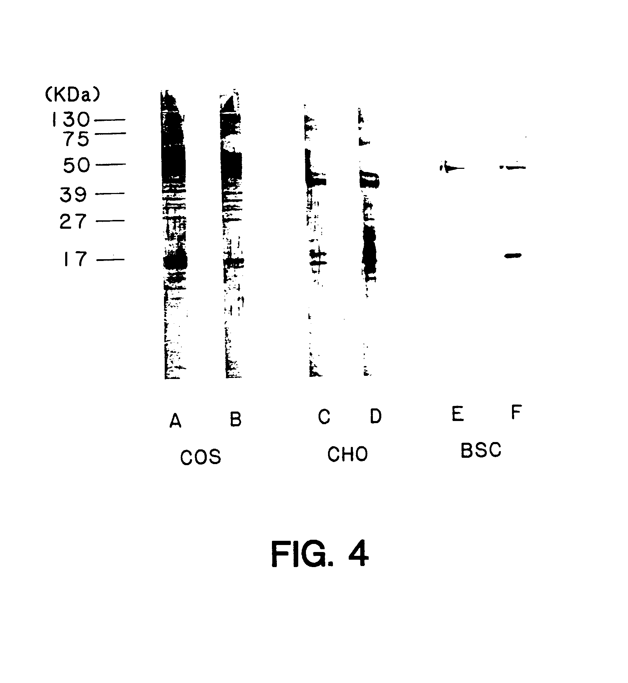

OP1 protein levels also may be measured by Western blots (immunoblots) using rabbit antisera against human OP1. Figure 4 is an immunoblot showing OP1 production in: COS cells - (A) pH717, (B) pH731; CHO cells - (C) pH754, (D) pH752; and BSC cells - (E) pH717 and (F) pW24.

Southern blots may be used to assess the state of integrated OP1 sequences and the extent of their copy number amplification. The copy number of excised plasmids in temperature-shifted BSC cells also can be determined using Southern blot analysis.

### II. PROTEIN PURIFICATION

The purification scheme developed to purify the recombinant osteogenic proteins of this invention is rapid and highly effective. The protocol involves three chromatographic steps (S-Sepharose, phenyl-Sepharose and C-18 HPLC), and produces OP1 of about 90% purity.

For a typical 2 L preparation of transfected BSC cells conditioned in 0.5% FCS, the total protein is 700 mg. The amount of OP1 in the media, estimated by western blot, is about 80 µg. OP1 media is diluted to 6M urea, 0.05M NaCl, 13mM HEPES, pH 7.0 and loaded onto an S-Sepharose column, which has attached sulfite groups and acts as a strong cation exchanger. OP1 binds to the column in low salt, and serum proteins are removed. The column is subsequently developed with two step salt elutions. The first elution (0.1M NaCl) removes contaminants and approximately 10% of the bound OP1. The remaining 90% of OP1 then is eluted in 6M urea, 0.3M NaCl, 20mM HEPES, pH 7.0.

Ammonium sulfate is added to the 0.3M NaCl fraction to obtain final solution conditions of 6M urea, 1M (NH₄)₂SO₄, 0.3M NaCl, 20mM HEPES, pH 7.0. The sample then is loaded onto a phenyl-Sepharose column (hydrophobic interaction chromatography). OP1 binds phenyl-Sepharose in the presence of high concentrations of a weak chaotropic salt (e.g., 1M (NH₄)₂SO₄). Once OP1 is bound, the column is developed with two step elutions using decreasing concentrations of ammonium sulfate. The first elution (containing 0.6M (NH₄)₂SO₄) primarily removes contaminants. The bound OP1 then is eluted with a 6M urea, 0.3M NaCl, 20MM HEPES, pH 7.0 buffer containing no ammonium sulfate.

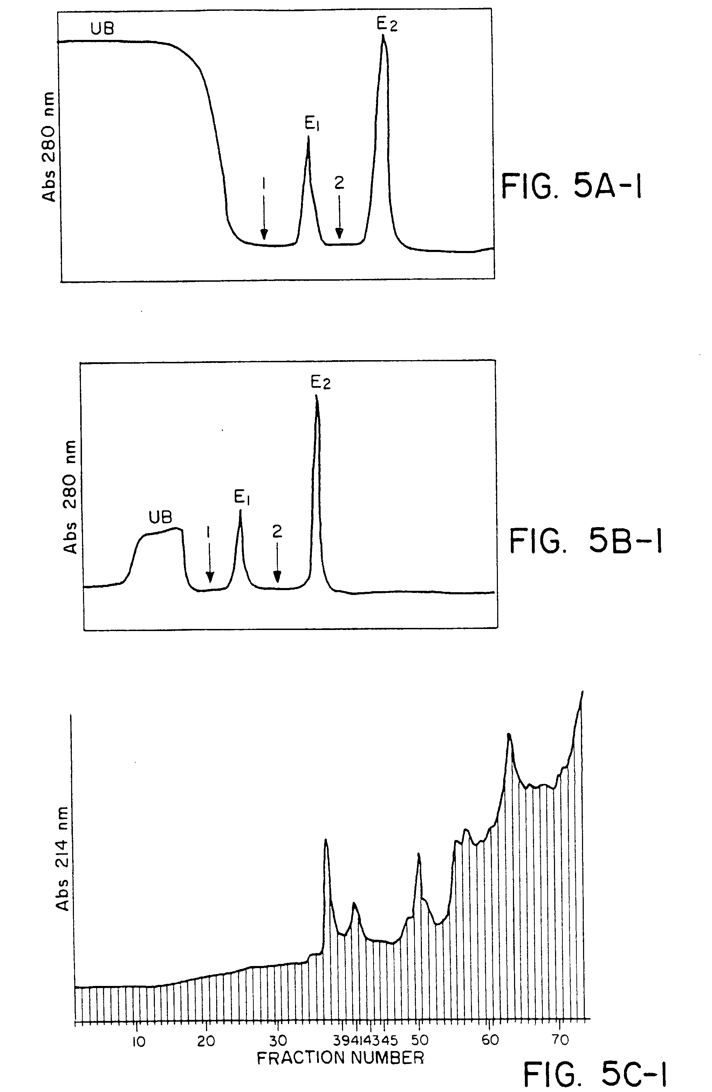

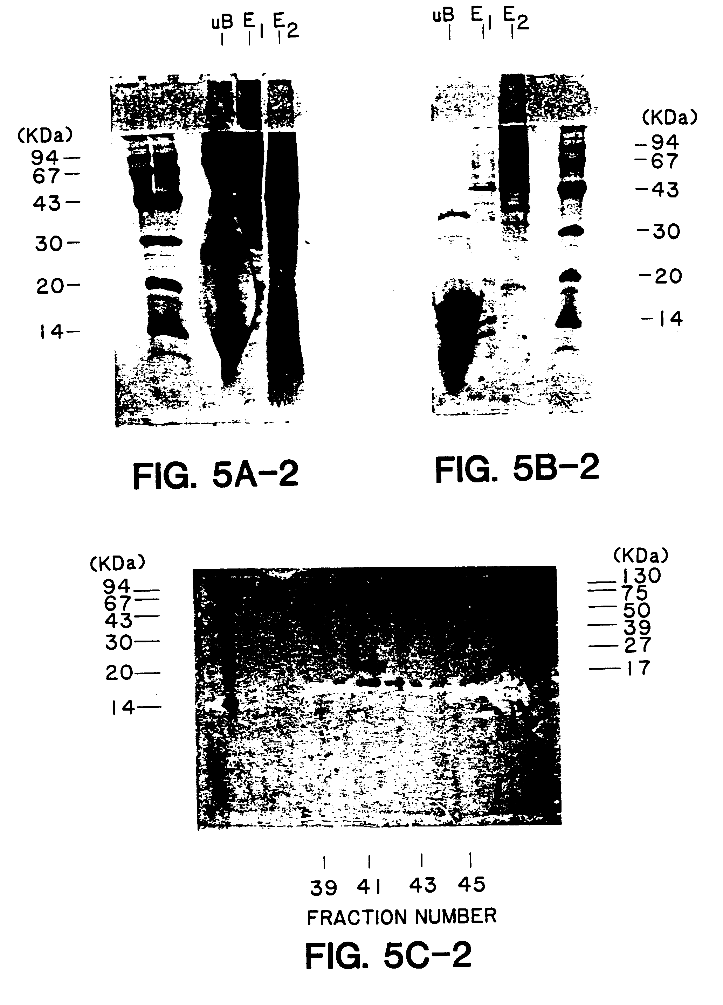

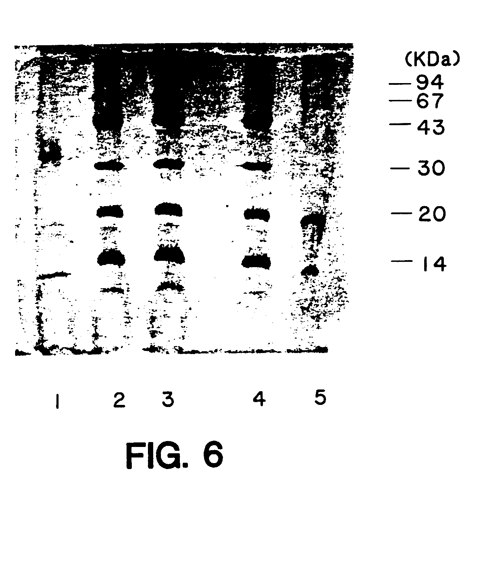

The OP1 eluted from the phenyl-Sepharose column is dialyzed against water, followed by 30% acetonitrile (0.1% TFA), and then applied to a C-18 reverse phase HPLC column. Figures 5A, B, and C are (1) chromatograms and (2) coomassie-stained SDS-PAGE gels of fractions after reduction with dithiothreitol (DTT) eluting from the (A) S-Sepharose, (B) phenyl-Sepharose, and (C) C-18 columns. Gel separation of oxidized and reduced OP1 samples show that the reduced subunit has an apparent molecular weight of about 18 kD, and the dimer has an apparent molecular weight of about 36 kD, as illustrated in Figure 6. The subunit size appears to be identical to that purified from COS cells, as well as that of the naturally-sourced bOP. The current protocol yields about 30 ug of OP1 for 2 L of conditioned media, a recovery of about 25%, as estimated by gel scanning.

An alternative chromatography protocol is to perform the S-Sepharose chromatography in the absence of 6 M urea. The bound proteins then are eluted with salt step elutions (e.g., 100-400 mM NaCl). Most of the OP1 is eluted with about 300 mM NaCl. Additional OP1 then can be eluted with 300 mM MaCl in the presence of 6M urea. The 6M urea elution also may be used in place of the non-urea elution to achieve maximum recovery in one step.

OP1 also will bind hydroxyapatite efficiently, but only in the absence of 6 M urea and at low phosphate concentrations (less than 5 mM phosphate). Bound OP1 can be removed from the column with a step elution of 1 mM to 0.5M phosphate (in 0.5 M NaCl, 50 mM Tris, pH 7.0). OP1 elutes at about 250 mM phosphate. Additionally, urea (6M) may be added during the elution step.

Other related chromatography methods also may be useful in purifying OP1 from eukaryotic cell culture systems. For example, heparin-Sepharose may be used in combination with the S-Sepharose column. Alternatively, Cu²⁺-immobilized metal-ion affinity chromatography (IMAC) will bind OP1 in a phosphate buffer (pH7.0) containing 6M urea.

### III. MATRIX PREPARATION

Practice of the invention requires the availability of bone, preferably mammalian bone, e.g., bovine. The bone is cleaned, demarrowed, delipidated, demineralized, reduced to particles of an appropriate size, extracted to remove soluble proteins, sterilized, and otherwise treated as disclosed herein to produce an implantable material useful in a variety of clinical settings.

Matrices of various shapes fabricated from the material of the invention may be implanted surgically for various purposes. Chief among these is to serve as a matrix for bone formation in various orthopedic, periodontal, and reconstructive procedures, as a sustained release carrier, or as a collagenous coating for implants. The matrix may be shaped as desired in anticipation of surgery or shaped by the physician or technician during surgery. Thus, the material may be used for topical, subcutaneous, intraperitoneal, or intramuscular implants; it may be shaped to span a nonunion fracture or to fill a bone defect. In bone formation or conduction procedures, the material is slowly absorbed by the body and is replaced by bone in the shape of or very nearly the shape of the implant.

Various growth factors, hormones, enzymes, therapeutic compositions, antibiotics, and other body treating agents also may be sorbed onto the carrier material and will be released over time when implanted as the matrix material is slowly absorbed. Thus, various known growth factors such as EGF, PDGF, IGF, FGF, TGF alpha, and TGF beta may be released in vivo. The material can be used to release chemotherapeutic agents, insulin, enzymes, or enzyme inhibitors.

Details of how to make and how to use the materials of the invention are disclosed below.

### 1. Preparation of Demineralized Bone

Demineralized bovine bone matrix is prepared by previously published procedures (Sampath and Reddi (1983) Proc. Natl. Acad. Sci. USA 80:6591-6595). Bovine diaphyseal bones (age 1-10 days) are obtained from a local slaughterhouse and used fresh. The bones are stripped of muscle and fat, cleaned of periosteum, demarrowed by pressure with cold water, dipped in cold absolute ethanol, and stored at -20°C. They are then dried and fragmented by crushing and pulverized in a large mill. Care is taken to prevent heating by using liquid nitrogen. The pulverized bone is milled to a particle size in the range of 70-850 µm, preferably 150 µm-420 µm, and is defatted by two washes of approximately two hours duration with three volumes of chloroform and methanol (3:1). The particulate bone is then washed with one volume of absolute ethanol and dried over one volume of anhydrous ether yielding defatted bone powder. The defatted bone powder is then demineralized by four successive treatments with 10 volumes of 0.5 N HCl at 4°C for 40 min. Finally, neutralizing washes are done on the demineralized bone powder with a large volume of water.

### 2. Guanidine Extraction

Demineralized bone matrix thus prepared is extracted with 5 volumes of 4 M guanidine-HCl, 50mM Tris-HCl, pH 7.0 for 16 hr. at 4°C. The suspension is filtered. The insoluble material is collected and used to fabricate the matrix. The material is mostly collagenous in nature. It is devoid of osteogenic or condrogenic activity.

### 3. Matrix Treatments

The major component of all bone matrices is Type-I collagen. In addition to collagen, demineralized bone extracted as disclosed above includes non-collagenous proteins which may account for 5% of its mass. In a xenogenic matrix, these noncollagenous components may present themselves as potent antigens, and may constitute immunogenic and/or inhibitory components. These components also may inhibit osteogenesis in allogenic implants by interfering with the developmental cascade of bone differentiation. It has been discovered that treatment of the matrix particles with a collagen fibril-modifying agent extracts potentially unwanted components from the matrix, and alters the surface structure of the matrix material.

The currently most preferred fibril modifying agent is a heated aqueous medium, most preferably water. Various amounts of delipidated, demineralized guanidine-extracted bone collagen is heated in water (1 g/30 ml) under constant stirring in a glass flask, water jacked, and maintained at a given temperature for 1 hour. In some instances the water may be replaced with 0.1M acetic acid to help "swell" the collagen before heating. The temperature employed is held constant at room temperature, and about 37°C, 45°C, 55°, 65°, 75°. After the heat treatment, the matrix is filtered and lyophilized and used for implant.

The effects of hot water treatment on morphology of the matrix material is apparent from a comparison of the photomicrographs in Figure 6 with those of Figure 1. Figure 6 illustrates the morphology of the successfully altered collagen surface treated at (a) 37°C, (b) 45°C, (c) 55°C and (d) 65°C. The photomicrographs of Figure 1 describe the morphology of untreated rat and bovine bone matrix (A and B, respectively). As is evident from the micrographs, the hot water treatment increases the degree of micropitting on the particle surface at least about 10-fold, as well as also substantially increasing particle's porosity (compare Figure 1B and 5C, 5D). This alteration of the matrix particle's morphology substantially increases the particle surface area. Careful measurement of the pore and micropit sizes reveals that hot aqueous medium treatment of the matrix particles yields particle pore and micropit diameters within the range of 1µm to 100µm.

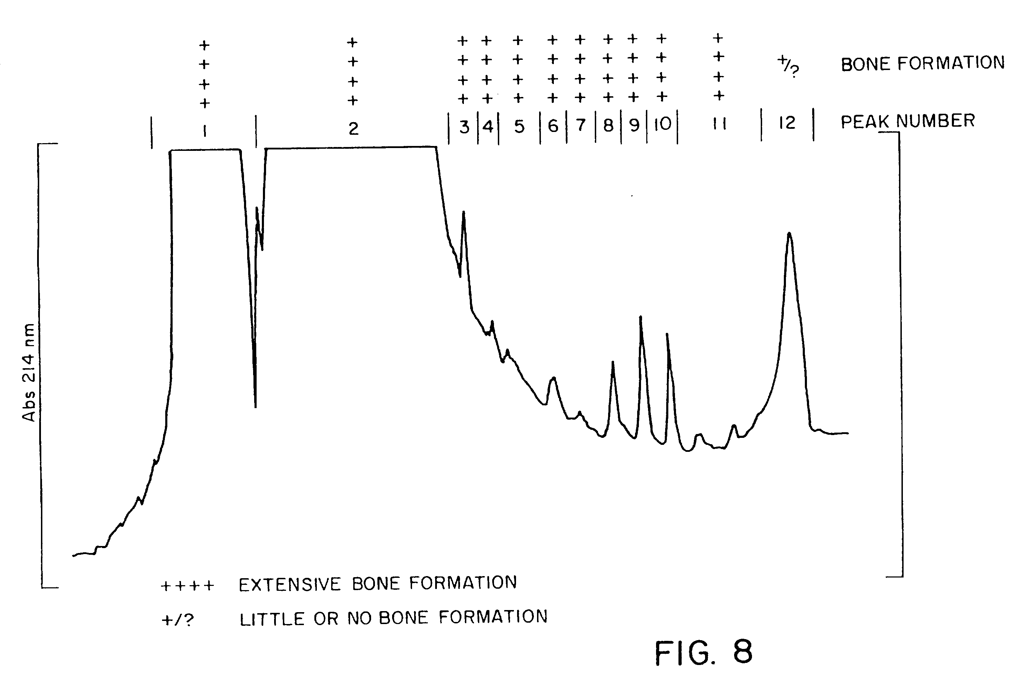

Characterization of the extract produced by the hot water treatment reveals that the treatment also may be removing component(s) whose association with the matrix may interfere with new bone formation in vivo. Figure 8 is a 214 nm absorbance tracing of the extract isolated from hot water treated bovine matrix, and indicates the effect of each peak (or fraction) on in vivo bone formation.

The extract from a large scale preparative run (100 g bovine matrix, hot water-treated) was collected, acidified with 0.1% TFA, and run on a C-18 HPLC column, using a Millipore Delta Prep Cartridge. Fractions were collected at 50 mL intervals at a flow rate of 25 ml/min. and pooled appropriately to isolate the individual peaks in the tracing. Each of these fractions then was implanted with recombinant OP1 and an appropriate rat matrix carrier (see infra), and its effect on bone formation activity measured. Fraction 12 alone appears to inhibit bone formation in allogenic implants. The inhibitory activity appears to be dose dependent. It is possible that the removal of the inhibitory component(s) present in this peak may be necessary to support osteogenic activity in xenogenic implants.

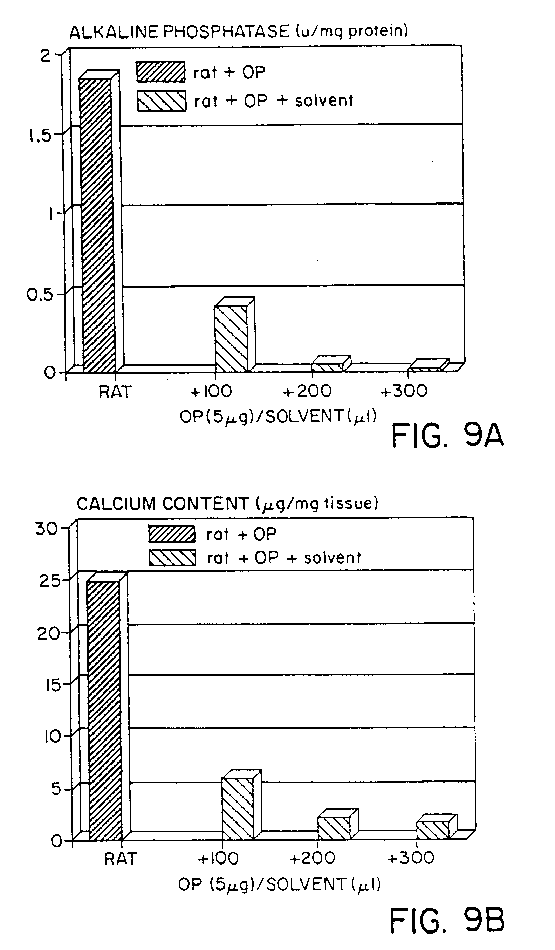

Figure 9 describes the influence of complete solvent extract from hot water-treated matrix on osteogenic activity as measured in 12-day implants, and determined by alkaline phosphatase activity and calcium content. Rat carrier matrix and OP1 implanted without any extract is used as a positive control. The solvent extract obtained from 100 grams of hot water-treated bovine matrix was evaporated and taken up in 6 M of 50% acetonitrile/0.1% TFA. 100-300 µl aliquots then were combined with known amounts of recombinant OP1, and 25 mg of rat matrix carrier, and assayed (see infra). The results clearly show the extract inhibits new bone formation in a dose dependent manner.

After contact with the fibril-modifying agent, the treated matrix is washed to remove any extracted components, following a form of the procedure set forth below:
1. Suspend in TBS (Tris-buffered saline) 1g/200 ml and stir at 4°C for 2 hrs; or in 6 M urea, 50 mM Tris-HCl, 500 mM NACl, pH 7.0 (UTBS) or water and stir at room temperature (RT) for 30 minutes (sufficient time to neutralize the pH);
2. Centrifuge and repeat wash step; and
3. Centrifuge; discard supernatant; water wash residue; and then lyophilize.

Other useful fibril-modifying agents include acids such as trifluoroacetic acid and hydrogen fluoride, and organic solvents such as dichloromethane, acetonitrile, isopropanol, and chloroform, as well as combinations of these agents. Matrix treatments using these other fibril-modifying agents, as well as a detailed physical analysis of the effect these fibril-modifying agents have on demineralized, guanidine-extracted bone collagen particles is disclosed in copending U.S. Patent Application No. 422,613, filed 10/17/89, the disclosure of which is hereby incorporated by reference.

The collagen matrix materials preferably take the form of a fine powder, insoluble in water, comprising nonadherent particles. It may be used simply by packing into the volume where new bone growth or sustained release is desired, held in place by surrounding tissue. Alternatively, the powder may be encapsulated in, e.g., a gelatin or polylactic acid coating, which is adsorbed readily by the body. The powder may be shaped to a volume of given dimensions and held in that shape by interadhering the particles using, for example, soluble, species biocompatible collagen. The material may also be produced in sheet, rod, bead, or other macroscopic shapes.

### IV. FABRICATION OF OSTEOGENIC DEVICE

The recombinant protein as set forth above, and other constructs, can be combined and dispersed in a suitable matrix preparation using any of the

### 1. Ethanol Precipitation

Matrix is added to osteogenic protein dissolved in guanidine-HCl. Samples are vortexed and incubated at a low temperature. Samples are then further vortexed. Cold absolute ethanol is added to the mixture which is then stirred and incubated. After centrifugation (microfuge, high speed) the supernatant is discarded. The matrix is washed with cold concentrated ethanol in water and then lyophilized.

### 2. Acetonitrile Trifluoroacetic Acid Lyophilization

In this procedure, osteogenic protein in an acetonitrile trifluroacetic acid (ACN/TFA) solution was added to the carrier material. Samples were vigorously vortexed many times and then lyophilized. Osteogenic protein was added in varying concentrations, and at several levels of purity. This method is currently preferred.

### 3. Urea Lyophilization

For those osteogenic proteins that are prepared in urea buffer, the protein is mixed with the matrix material, vortexed many times, and then lyophilized. The lyophilized material may be used "as is" for implants.

### 4. Buffered Saline Lyophilization

OP preparations in physiological saline may also be vortexed with the matrix and lyophilized to produce osteogenically active material.

These procedures also can be used to adsorb other active therapeutic drugs, hormones, and various bioactive species for sustained release purposes.

### V. BIOASSAY

The functioning of the various matrices can be evaluated with an in vivo rat bioassay. Studies in rats show the osteogenic effect in an appropriate matrix to be dependent on the dose of osteogenic protein dispersed in the matrix. No activity is observed if the matrix is implanted alone. Demineralized, guanidine extracted xenogenic bone matrix materials of the type described in the literature are ineffective as a carrier, fail to induce bone, and produce an inflammatory and immunological response when implanted unless treated as disclosed above. Many of the allogenic matrix materials also are ineffective as carriers. The following sets forth various procedures for preparing osteogenic devices from control and matrix materials prepared as set forth above, and for evaluating their osteogenic utility.

### Implantation

The bioassay for bone induction as described by Sampath and Reddi (Proc. Natl. Acad. Sci. USA (1983) 80: 6591-6595), herein incorporated by reference, may be used to monitor endochondral bone differentiation activity. This assay consists of implanting the bovine test samples xenogenically in subcutaneous sites in recipient rats under ether anesthesia. Male Long-Evans rats, aged 28-32 days, were used. A vertical incision (1 cm) is made under sterile conditions in the skin over the thoraic region, and a pocket is prepared by blunt dissection. Approximately 25 mg of the test sample is implanted deep into the pocket and the incision is closed with a metallic skin clip. The day of implantation is designated as day of the experiment. Implants were removed on day 12. The heterotropic site allows for the study of bone induction without the possible ambiguities resulting from the use of orthotropic sites.

### Cellular Events

Successful implants exhibit a controlled progression through the stages of matrix induced endochondral bone development including: (1) transient infiltration by polymorphonuclear leukocytes on day one; (2) mesenchymal cell migration and proliferation on days two and three; (3) chondrocyte appearance on days five and six; (4) cartilage matrix formation on day seven; (5) cartilage calcification on day eight; (6) vascular invasion, appearance of osteoblasts, and formation of new bone on days nine and ten; (7) appearance of osteoblastic and bone remodeling and dissolution of the implanted matrix on days twelve to eighteen; and (8) hematopoietic bone marrow differentiation in the ossicle on day twenty-one. The results show that the shape of the new bone conforms to the shape of the implanted matrix.

### Histological Evaluation

Histological sectioning and staining is preferred to determine the extent of osteogenesis in the implants. Implants are fixed in Bouins Solution, embedded in paraffin, and cut into 6-8 µm sections. Staining with toluidine blue or hemotoxylin/eosin demonstrates clearly the ultimate development of endochondral bone. Twelve day implants are usually sufficient to determine whether the implants contain newly induced bone.

### Biological Markers

Alkaline phosphatase activity may be used as a marker for osteogenesis. The enzyme activity may be determined spectrophotometrically after homogenization of the implant. The activity peaks at 9-10 days in vivo and thereafter slowly declines. Implants showing no bone development by histology have little or no alkaline phosphatase activity under these assay conditions. The assay is useful for quantitation and obtaining an estimate of bone formation quickly after the implants are removed from the rat. Alternatively, the amount of bone formation can be determined by measuring the calcium content of the implant.

### Results

OP1 from different cell sources and purified to different extents (1-5% pure to 30-90% pure) were tested for osteogenic activity in vivo as set forth above using matrices of approximately 25 mg. Table III below shows the histology score for OP1 expressed in all three cell types.

**TABLE III**

| Mammalian Cells | OP1 Subunit | Protein Concentration † (ng) | Histology Score (%) |
|---|---|---|---|
| BSC40-tsA58 | 18kDa* | 32.5 | 50 |
| | | 65.0 | 40 |
| | | 130.0 | 80 |
| | | 260.0 | 100 |
| | | | |
| | 16 kDa⁺ | 12.5 | 20 |
| | | 25.0 | 50 |
| | | 50.0 | 80 |
| | | 100.0 | 100 |
| | | 200.0 | 100 |
| | | | |
| CHO | 16-20 kDa^{s} | 50.0 | 90 |
| | | 100.0 | 90 |
| | | 200.0 | 100 |
| COS | 18 kDa^{s} | 25.0 | 10 |
| | | 50.0 | 30 |
| | | 100.0 | 90 |
| | | 200.0 | 90 |

| | | | |
|---|---|---|---|
| 10-30%: moderate bone formation 30-80%: extensive bone formation above 80%: showed sign of hemopoietic bone marrow recruitment. * 70-90% pure | | | |
| + 30-40% pure | | | |
| ^{s} less than 5% pure | | | |
| † estimated by immunoblots or gel scanning | | | |

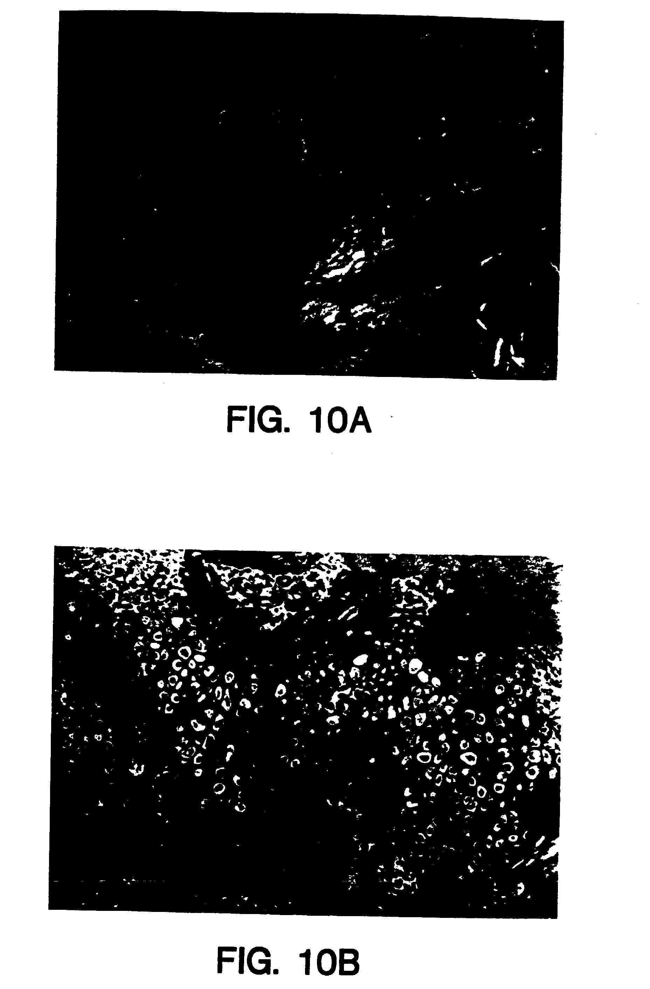

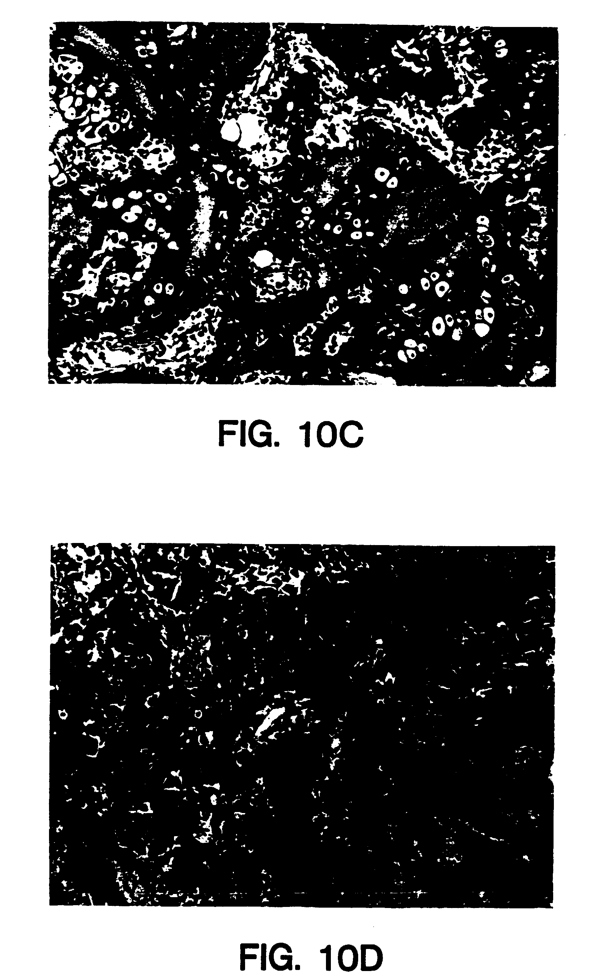

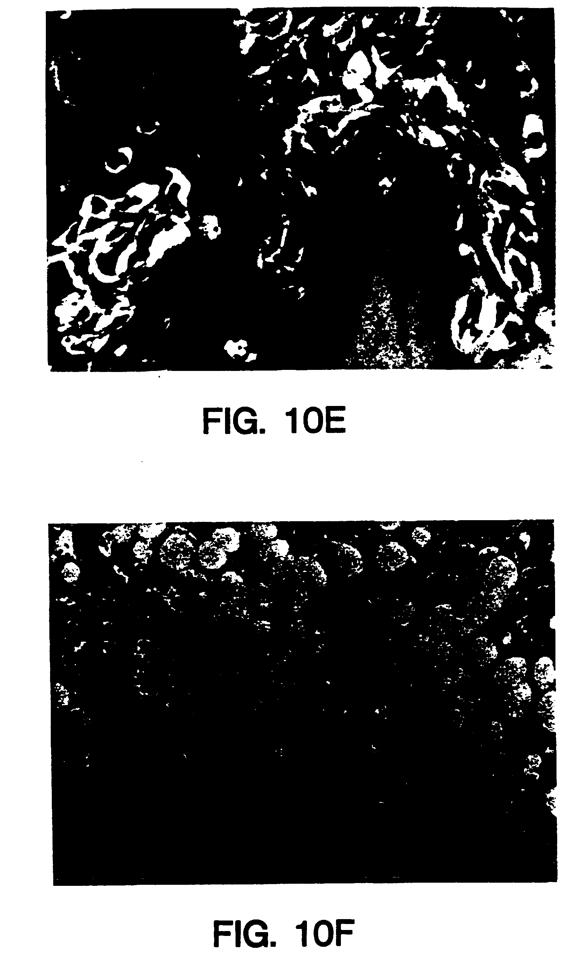

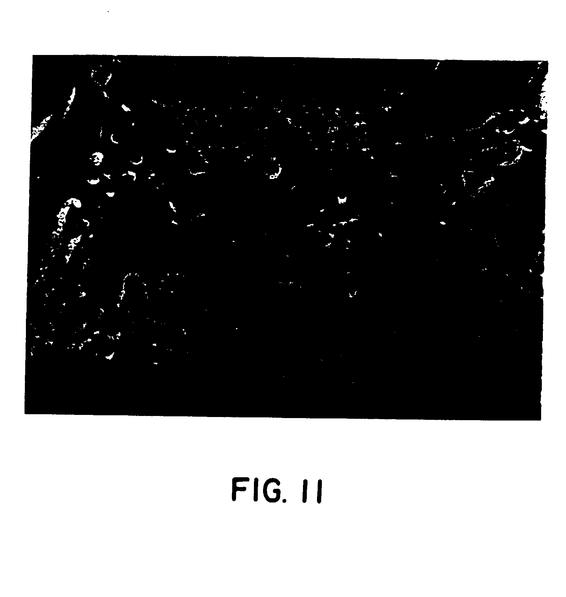

The histology scores detailed in Table III show that OP1 is active regardless of cell source, and that the activity mimics that of native bovine OP. The bone-inducing activity is highly reproducible and dose dependent. Further evidence of the bone-forming activity of recombinant OP1 is provided in the photomicrographs of Figures 10 and 11.

Figure 10A-F are photomicrographs recording the histology of allogenic implants using recombinant OP1 expressed from COS, BSC, and COS cells. The micrographs (magnified 220X), provide graphic evidence of the full developmental cascade induced by the osteogenic proteins of this invention, confirming that recombinantly produced OP1 alone is sufficient to induce endochondral bone formation, when implanted in association with a matrix. As evidenced in Figure 10A, allogenic implants that do not contain OP1 show no new bone formation at 12 days' post implant. Only the implanted bone matrix (m) and surrounding mesenchyme are seen. Conversely, implants containing OP1 already show evidence of extensive chodrogenesis by 7 days post implant (Fig. 10B, 500 ng BSC-produced protein, 30% pure). Here, newly formed cartilage cells, chrondroblasts (Cb) and chondrocytes (Cy) are in close contact with the matrix (m). By 9 days post implant endochondral bone differentiation, cartilage calcification, hypertrophy of chondrocytes, vascular invasion, and the onset of new bone formation are all evident (Fig. 10C, 220 ng COS-produced protein, approx. 5% pure). Invading capillaries (c) and the appearance of basophilic osteoblasts (indicated by arrows) near the vascular endothelium are particularly evident. By 12 days post implant extensive bone formation and remodeling has occurred (Fig. 10D (220X), and 10E (400X), CHO-produced protein, approx. 60% pure). The newly formed bone laid down by osteoblasts is being remodeled by multinucleated osteoclasts (Oc), and the implanted matrix is being resorbed and replaced by remodeled bone. Bone marrow recruitment in the newly formed ossicles is also evident. Finally, hematopoietic bone marrow differentiation within the ossicles can be seen by 22 days' post implant (Fig. 10F, 500 ng BSC-produced protein, 30% pure). By this time most of the implanted matrix (m) has been resorbed and is occupied by newly-formed bone containing ossicles filled with bone marrow elements including erythrocytic and granulocytic series and megakaryocytes. Similar histological observations have been made for implants incorporating greater than 90% pure OP1 preparations.

Figure 11 is a photomicrograph showing the histology at 12 days post implant for a xenogenic implant using hot water-treated bovine matrix and OP1 (BSC-produced). The recruitment of hematopoietic bone marrow elements is evident in the photomicrograph, showing that the bone forming activity of xenogenic implants with OP1 parallels that of allogenic implants (compare Figure 11 with Figures 10D and 10E).

The cellular events exhibited by the OP1 matrix implants and evidenced in Figures 10 and 11 truly mimics the endochondral bone differentiation that occurs during the foetal development. Although endochondral bone differentiation has been the predominant route, there is also evidence for intra-membraneous bone formation at the outer surface of the implant.

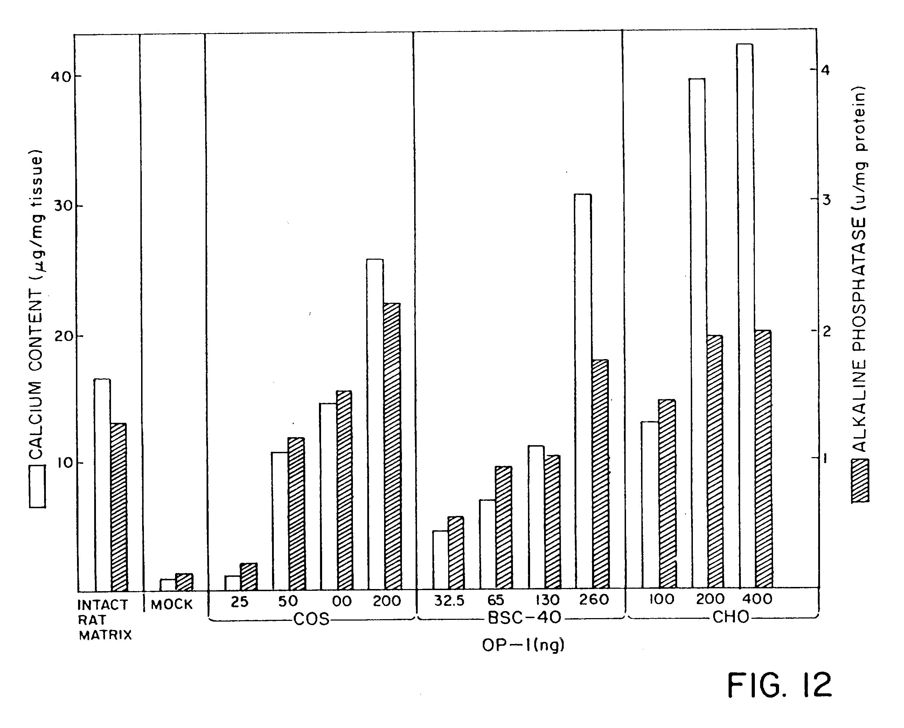

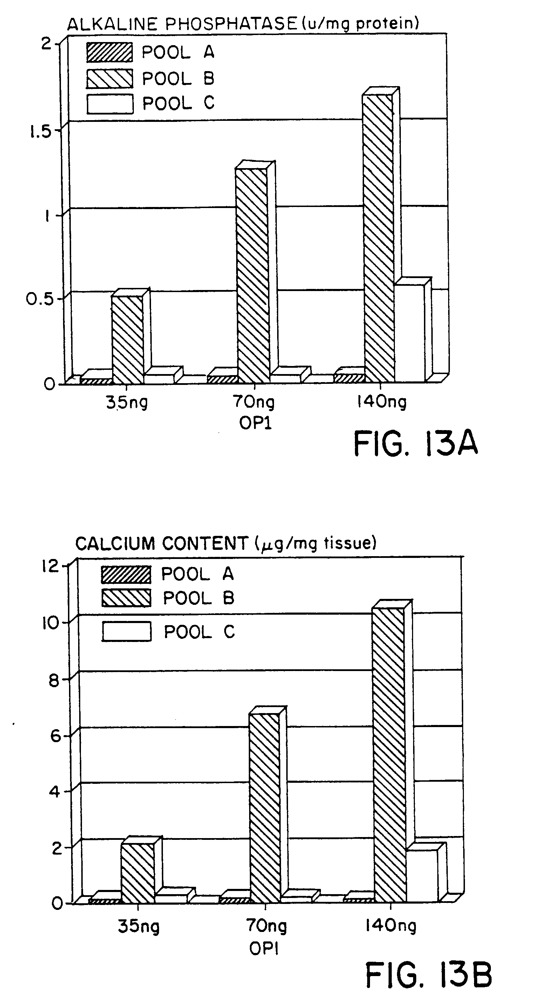

Figures 12 and 13 describe the dose dependence of osteogenic activity for 12-day implants, as determined by specific activity of alkaline phosphatase and calcium content of allogenic implants (Figure 12) and xenogenic implants of this invention (Figure 13). In all cases, OP1 protein concentration (quantitated by immuno blot staining or by gel scanning), is represented in nanograms. In each case, bone inducing activity is specific to OP1 in a dose dependent manner in all cells.

The invention may be embodied in other specific forms. The present embodiments are therefore to be considered in all respects as illustrative and not restrictive, the scope of the invention being indicated by the appended claims rather than by the foregoing description.

### SEQUENCE LISTING

### (1) GENERAL INFORMATION:

(i) APPLICANT:
   Oppermann, Hermann
   Kuberasampath, Thangavel
   Rueger, David C.
   Ozkaynak, Engin
   Pang, Roy H.L.
(ii) TITLE OF INVENTION: Osteogenic Devices
(iii) NUMBER OF SEQUENCE: 7
(iv) CORRESPONDENCE ADDRESS:
   (A) ADDRESSEE: Lahive & Cockfield
   (B) STREET: 60 State Street
   (C) CITY: Boston
   (D) STATE: Massachusetts
   (E) COUNTRY: U.S.A.
   (F) ZIP: 02109
(v) COMPUTER READABLE FORM:
   (A) MEDIUM TYPE: Diskette, 3.5 inch, 720kb storage
   (B) COMPUTER: IBM XT
   (C) OPERATING SYSTEM: DOS 3.30
   (D) SOFTWARE: Word Perfect 5.0
(vi) CURRENT APPLICATION DATA:
   (B) FILING DATE: 20-Aug-90
(vii) PRIOR APPLICATION DATA:
   (A) APPLICATION NUMBER: US 422,699
   (B) FILING DATE: 17-Oct-89
   (C) APPLICATION NUMBER: US 483,913
   (D) FILING DATE: 22-Feb-89

### (2) INFORMATION FOR SEQ ID NO:1:

(i) SEQUENCE CHARACTERISTICS:
   (A) LENGTH: 139 amino acids
   (B) TYPE: amino acid
   (D) TOPOLOGY: linear
(ii) MOLECULE TYPE: protein
(xi) SEQUENCE DESCRIPTION: SEQ ID NO:1:

### (2) INFORMATION FOR SEQ ID NO:2:

(i) SEQUENCE CHARACTERISTICS:
   (A) LENGTH: 132 amino acids
   (B) TYPE: amino acid
   (D) TOPOLOGY: linear
(ii) MOLECULE TYPE: protein
(xi) SEQUENCE DESCRIPTION: SEQ ID NO:2:

### (2) INFORMATION FOR SEQ ID NO:3:

(i) SEQUENCE CHARACTERISTICS:
   (A) LENGTH: 119 amino acids
   (B) TYPE: amino acid
   (D) TOPOLOGY: linear
(ii) MOLECULE TYPE: protein
(xi) SEQUENCE DESCRIPTION: SEQ ID NO:3:
   Leu Arg Met Ala Asn Val Ala Glu Asn
   1 5
   Ser Ser Ser Asp Gln Arg Gln Ala Cys
   10 15
   Lys Lys His Glu Leu Tyr Val Ser Phe
   20 25
   Arg Asp Leu Gly Trp Gln Asp Trp Ile
   30 35
   Ile Ala Pro Glu Gly Tyr Ala Ala Tyr
   40 45
   Tyr  Cys  Glu  Gly  Glu  Cys  Ala  Phe  Pro
                        50
   Leu  Asn  Ser  Tyr  Met  Asn  Ala  Thr  Asn
    55                       60
   His  Ala  Ile  Val  Gln  Thr  Leu  Val  His
         65                       70
   Phe  Ile  Asn  Pro  Glu  Thr  Val  Pro  Lys
              75                       80
   Pro  Cys  Cys  Ala  Pro  Thr  Gln  Leu  Asn
                   85                       90
   Ala  Ile  Ser  Val  Leu  Tyr  Phe  Asp  Asp
                        95
   Ser  Ser  Asn  Val  Ile  Leu  Lys  Lys  Tyr
   100                      105
   Arg  Asn  Met  Val  Val  Arg  Ala  Cys  Gly
        110                      115
   Cys  His

### (2) INFORMATION FOR SEQ ID NO:4:

(i) SEQUENCE CHARACTERISTICS:
   (A) LENGTH: 117 amino acids
   (B) TYPE: amino acid
   (D) TOPOLOGY: linear
(ii) MOLECULE TYPE: protein
(xi) SEQUENCE DESCRIPTION: SEQ ID NO:4:

### (2) INFORMATION FOR SEQ ID NO:5:

(i) SEQUENCE CHARACTERISTICS:
   (A) LENGTH: 116 amino acids
   (B) TYPE: amino acid
   (D) TOPOLOGY: linear
(ii) MOLECULE TYPE: protein
(xi) SEQUENCE DESCRIPTION: SEQ ID NO:5:
   Ala Asn Val Ala Glu Asn
   1               5
   Ser Ser Ser Asp Gln Arg Gln Ala Cys
               10                  15
   Lys Lys His Glu Leu Tyr Val Ser Phe
                   20
   Arg Asp Leu Gly Trp Gln Asp Trp Ile
    25                  30
   Ile Ala Pro Glu Gly Tyr Ala Ala Tyr
        35                  40
   Tyr Cys Glu Gly Glu Cys Ala Phe Pro
            45                  50
   Leu Asn Ser Tyr Met Asn Ala Thr Asn
                55                  60
   His Ala Ile Val Gln Thr Leu Val His
                    65
   Phe Ile Asn Pro Glu Thr Val Pro Lys
    70                  75
   Pro Cys Cys Ala Pro Thr Gln Leu Asn
        80                  85
   Ala Ile Ser Val Leu Tyr Phe Asp Asp
            90                  95
   Ser Ser Asn Val Ile Leu Lys Lys Tyr
               100                 105
   Arg Asn Met Val Val Arg Ala Cys Gly
                   110
   Cys His
   115

### (2) INFORMATION FOR SEQ ID NO:6:

(i) SEQUENCE CHARACTERISTICS:
   (A) LENGTH: 114 amino acids
   (B) TYPE: amino acid
   (D) TOPOLOGY: linear
(ii) MOLECULE TYPE: protein
(xi) SEQUENCE DESCRIPTION: SEQ ID NO:6:
   Val Ala Glu Asn Ser Ser Ser Asp Gln
     1                 5
   Arg Gln Ala Cys Lys Lys His Glu Leu
    10                 15
   Tyr Val Ser Phe Arg Asp Leu Gly Trp
        20                 25
   Gln Asp Trp Ile Ile Ala Pro Glu Gly
            30                 35
   Tyr Ala Ala Tyr Tyr Cys Glu Gly Glu
                40                 45
   Cys Ala Phe Pro Leu Asn Ser Tyr Met
                    50
   Asn Ala Thr Asn His Ala Ile Val Gln
    55                 60
   Thr Leu Val His Phe Ile Asn Pro Glu
        65                 70
   Thr Val Pro Lys Pro Cys Cys Ala Pro
            75                 80
   Thr Gln Leu Asn Ala Ile Ser Val Leu
                85                 90
   Tyr Phe Asp Asp Ser Ser Asn Val Ile
                    95
   Leu Lys Lys Tyr Arg Asn Met Val Val
   100                 105
   Arg Ala Cys Gly Cys His
       110

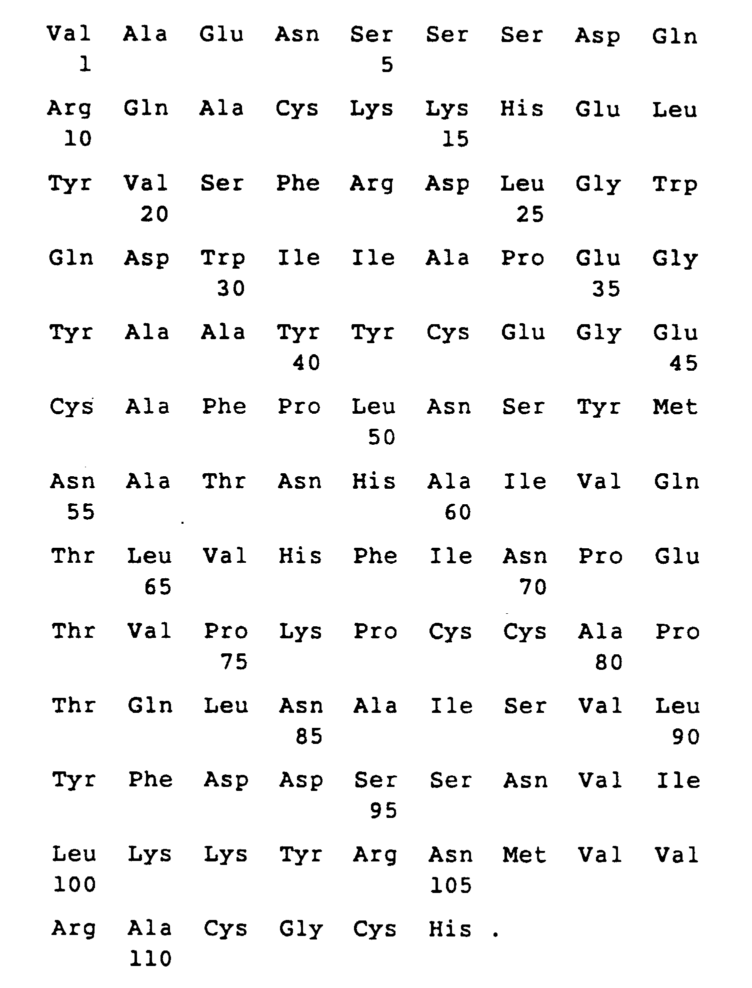

### (2) INFORMATION FOR SEQ ID NO:7:

(i) SEQUENCE CHARACTERISTICS:
   (A) LENGTH: 1822 base pairs
   (B) TYPE: nucleic acid
   (C) STRANDEDNESS: single
   (D) TOPOLOGY: linear
(ii) MOLECULE TYPE: cDNA to mRNA
(iii) HYPOTHETICAL: no
(iv) ANTI-SENSE: no
(vi) ORIGINAL SOURCE:
   (A) ORGANISM: Bovinae
   (F) TISSUE TYPE: bone
(vii) IMMEDIATE SOURCE:
   (A) LIBRARY: human placenta
(xi) SEQUENCE DESCRIPTION: SEQ ID NO:7:
   GGTGCGGGCC CGGAGCCCGG AGCCCGGGTA GCGCGTAGAG      40
   CCGGCGCG ATG CAC GTG CGC TCA CTG CGA GCT GCG     75
            Met His Val Arg Ser Leu Arg Ala Ala
             1               5
   GCG CCG CAC AGC TTC GTG GCG CTC TGG GCA CCC     108
   Ala Pro His Ser Phe Val Ala Leu Trp Ala Pro
    10                 15                  20
   CTG TTC CTG CTG CGC TCC GCC CTG GCC GAC TTC     141
   Leu Phe Leu Leu Arg Ser Ala Leu Ala Asp Phe
                   25                  30
   AGC CTG GAC AAC GAG GTG CAC TCG AGC TTC ATC     174
   Ser Leu Asp Asn Glu Val His Ser Ser Phe Ile
               35                  40
   CAC CGG CGC CTC CGC AGC CAG GAG CGG CGG GAG     207
   His Arg Arg Leu Arg Ser Gln Glu Arg Arg Glu
           45                  50
   ATG CAG CGC GAG ATC CTC TCC ATT TTG GGC TTG     240
   Met Gln Arg Glu Ile Leu Ser Ile Leu Gly Leu
       55                  60
   CCC CAC CGC CCG CGC CCG CAC CTC CAG GGC AAG     273
   Pro His Arg Pro Arg Pro His Leu Gln Gly Lys
    65                 70                  75
   CAC AAC TCG GCA CCC ATG TTC ATG CTG GAC CTG 306
   His Asn Ser Ala Pro Met Phe Met Leu Asp Leu
   80 85
   TAC AAC GCC ATG GCG GTG GAG GAG GGC GGC GGG 339
   Tyr Asn Ala Met Ala Val Glu Glu Gly Gly Gly
   90 95
   CCC GGC GGC CAG GGC TTC TCC TAC CCC TAC AAG 372
   Pro Gly Gly Gln Gly Phe Ser Tyr Pro Tyr Lys
   100 105
   GCC GTC TTC AGT ACC CAG GGC CCC CCT CTG GCC 405
   Ala Val Phe Ser Thr Gln Gly Pro Pro Leu Ala
   110 115
   AGC CTG CAA GAT AGC CAT TTC CTC ACC GAC GCC 438
   Ser Leu Gln Asp Ser His Phe Leu Thr Asp Ala
   120 125 130
   GAC ATG GTC ATG AGC TTC GTC AAC CTC GTG GAA 471
   Asp Met Val Met Ser Phe Val Asn Leu Val Glu
   135 140
   CAT GAC AAG GAA TTC TTC CAC CCA CGC TAC CAC 504
   His Asp Lys Glu Phe Phe His Pro Arg Tyr His
   145 150
   CAT CGA GAG TTC CGG TTT GAT CTT TCC AAG ATC 537
   His Arg Glu Phe Arg Phe Asp Leu Ser Lys Ile
   155 160
   CCA GAA GGG GAA GCT GTC ACG GCA GCC GAA TTC 570
   Pro Glu Gly Glu Ala Val Thr Ala Ala Glu Phe
   165 170
   CGG ATC TAC AAG GAC TAC ATC CGG GAA CGC TTC 603
   Arg Ile Tyr Lys Asp Tyr Ile Arg Glu Arg Phe
   175 180 185
   GAC AAT GAG ACG TTC CGG ATC AGC GTT TAT CAG 636
   Asp Asn Glu Thr Phe Arg Ile Ser Val Tyr Gln
   190 195
   GTG CTC CAG GAG CAC TTG GGC AGG GAA TCG GAT 669
   Val Leu Gln Glu His Leu Gly Arg Glu Ser Asp
               200                 205
   CTC TTC CTG CTC GAC AGC CGT ACC CTC TGG GCC    702
   Leu Phe Leu Leu Asp Ser Arg Thr Leu Trp Ala
           210                 215
   TCG GAG GAG GGC TGG CTG GTG TTT GAC ATC ACA    735
   Ser Glu Glu Gly Trp Leu Val Phe Asp Ile Thr
       220                 225
   GCC ACC AGC AAC CAC TGG GTG GTC AAT CCG CGG    768
   Ala Thr Ser Asn His Trp Val Val Asn Pro Arg
   230                 235                 240
   CAC AAC CTG GGC CTG CAG CTC TCG GTG GAG ACG    801
   His Asn Leu Gly Leu Gln Leu Ser Val Glu Thr
                   245                 250
   CTG GAT GGG CAG AGC ATC AAC CCC AAG TTG GCG    834
   Leu Asp Gly Gln Ser Ile Asn Pro Lys Leu Ala
               255                 260
   GGC CTG ATT GGG CGG CAC GGG CCC CAG AAC AAG    867
   Gly Leu Ile Gly Arg His Gly Pro Gln Asn Lys
           265                 270
   CAG CCC TTC ATG GTG GCT TTC TTC AAG GCC ACG    900
   Gln Pro Phe Met Val Ala Phe Phe Lys Ala Thr
       275                 280
   GAG GTC CAC TTC CGC AGC ATC CGG TCC ACG GGG    933
   Glu Val His Phe Arg Ser Ile Arg Ser Thr Gly
   285                 290                 295
   AGC AAA CAG CGC AGC CAG AAC CGC TCC AAG ACG    966
   Ser Lys Gln Arg Ser Gln Asn Arg Ser Lys Thr
                   300                 305
   CCC AAG AAC CAG GAA GCC CTG CGG ATG GCC AAC    999
   Pro Lys Asn Gln Glu Ala Leu Arg Met Ala Asn
               310                 315
   GTG GCA GAG AAC AGC AGC AGC GAC CAG AGG CAG   1032
   Val Ala Glu Asn Ser Ser Ser Asp Gln Arg Gln
           320                 325
   GCC TGT AAG AAG CAC GAG CTG TAT GTC AGC TTC   1065
   Ala Cys Lys Lys His Glu Leu Tyr Val Ser Phe
       330                 335
   CGA GAC CTG GGC TGG CAG GAC TGG ATC ATC GCG   1098
   Arg Asp Leu Gly Trp Gln Asp Trp Ile Ile Ala
   340                 345                 350
   CCT GAA GGC TAC GCC GCC TAC TAC TGT GAG GGG   1131
   Pro Glu Gly Tyr Ala Ala Tyr Tyr Cys Glu Gly
                   355                 360
   GAG TGT GCC TTC CCT CTG AAC TCC TAC ATG AAC   1164
   Glu Cys Ala Phe Pro Leu Asn Ser Tyr Met Asn
               365                 370
   GCC ACC AAC CAC GCC ATC GTG CAG ACG CTG GTC   1197
   Ala Thr Asn His Ala Ile Val Gln Thr Leu Val
           375                 380
   CAC TTC ATC AAC CCG GAA ACG GTG CCC AAG CCC   1230
   His Phe Ile Asn Pro Glu Thr Val Pro Lys Pro
       385                 390
   TGC TGT GCG CCC ACG CAG CTC AAT GCC ATC TCC   1263
   Cys Cys Ala Pro Thr Gln Leu Asn Ala Ile Ser
   395                 400                 405
   GTC CTC TAC TTC GAT GAC AGC TCC AAC GTC ATC   1296
   Val Leu Tyr Phe Asp Asp Ser Ser Asn Val Ile
                   410                 415
   CTG AAG AAA TAC AGA AAC ATG GTG GTC CGG GCC   1329
   Leu Lys Lys Tyr Arg Asn Met Val Val Arg Ala
               420                 425
   TGT GGC TGC CAC TAGCTCCTCC GAGAATTCAG         1361
   Cys Gly Cys His
           430
   ACCCTTTGGG GCCAAGTTTT TCTGGATCCT CCATTGCTCG   1401
   CCTTGGCCAG GAACCAGCAG ACCAACTGCC TTTTGTGAGA   1441
   CCTTCCCCTC CCTATCCCCA ACTTTAAAGG TGTGAGAGTA   1481
   TTAGGAAACA TGAGCAGCAT ATGGCTTTTG ATCAGTTTTT   1521
   CAGTGGCAGC ATCCAATGAA CAAGATCCTA CAAGCTGTGC   1561
   AGGCAAAACC TAGCAGGAAA AAAAAACAAC GCATAAAGAA   1601
   AAATGGCCGG GCCAGGTCAT TGGCTGGGAA GTCTCAGCCA   1641
   TGCACGGACT CGTTTCCAGA GGTAATTATG AGCGCCTACC   1681
   AGCCAGGCCA CCCAGCCGTG GGAGGAAGGG GGCGTGGCAA   1721
   GGGGTGGGCA CATTGGTGTC TGTGCGAAAG GAAAATTGAC   1761
   CCGGAAGTTC CTGTAATAAA TGTCACAATA AAACGAATGA   1801
   ATGAAAAAAA AAAAAAAAAA A                       1822

## Claims

1. Isolated prepro- or pro-OP1 polypeptide.

2. A composition comprising the isolated polypeptide of claim 1.

3. An osteogenic device comprising the isolated polypeptide of claim 1 or the composition of claim 2 dispersed in a matrix, the polypeptide being properly dimerized and folded.

4. Isolated DNA encoding prepro- or pro-OP1.

5. DNA according to claim 4, said DNA comprising:
(a) the nucelotide sequence corresponding to positions 49-1341 of Seq. ID. No. 7 (Fig. 2); or
(b) a sequence encoding the amino acid sequence corresponding to residues 1-431 of Seq. ID. No. 7 (Fig. 2); or
(c) a DNA sequence encoding a prepro- or pro-OP1 mutein.

6. DNA according to claim 5, said DNA comprising the nucelotide sequence corresponding to positions 1-1822 of Seq. ID No. 7 (Fig. 2).

7. The DNA of any one of claims 4 to 6 which is cDNA, genomic DNA or DNA constructed from oligonucleotides.

8. A vector comprising the DNA of any one of claims 4 to 7 in operative association with an expression control sequence therefor.

9. A host cell transformed with the vector of claim 8 or the DNA of any one of claims 5-7.

10. The host cell of claim 9 which is prokaryotic or eukaryotic, for example a mammalian, Saccharomyces or E. coli cell.

11. An osteogenic protein produced by expression from a mammalian host cell transformed with the DNA of claim 5 and competent to induce cartilage and bone formation in a mammal.

12. A method for producing an osteogenic protein capable of inducing cartilage and bone formation in a mammal, the method comprising the steps of:
(a) culturing in a suitable culture medium the host cell of claim 9 or 10, and
(b) recovering, isolating and purifying said protein from said culture medium.

13. A process for producing OP1 protein comprising the step of culturing a cell containing DNA encoding pro- or prepro-OP1 under conditions such that said DNA is expressed to produce an OP1 protein which undergoes maturation by post-translational proteolytic cleavage and secretion from the cell.

14. The process of claim 13 wherein the cell is a mammalian host cell, the host cell is cultured in a culture medium and the DNA encodes amino acids 1 to 431 of Sequence ID. No. 7 (or an amino acid sequence related thereto or mutated therefrom), further comprising the step of recovering said protein from said culture medium.

15. A process for producing an active osteogenic protein composition comprising the step of truncating mature OP1.

16. Mature OP1 as secreted from a mammalian host cell culture upon or after expression of the sequence shown in Fig. 2.

17. An osteogenic protein competent to induce cartilage and bone formation in a mammal, obtainable by the steps of:
(a) culturing in a suitable culture a mammalian host cell transformed with a DNA sequence encoding amino acids 1 to 431 of Sequence ID. No. 7 (or a DNA sequence related thereto or mutated therefrom), and
(b) recovering said protein from said culture medium.

18. Active osteogenic protein comprising an OP1/BMP2 heterodimer.

19. The protein of claim 18 wherein the heterodimer is joined by disulphide bonds or otherwise associated.

20. A process for producing a composition comprising active heterodimeric osteogenic protein comprising the step of folding and oxidizing two or more monomeric species within a eukaryotic cell (e.g. a mammalian cell), at least one of the monomeric species being OP1.

21. A process for producing a composition comprising active heterodimeric osteogenic protein comprising the steps of:
(a) expressing individual monomeric species; and
(b) oxidizing and refolding each of the monomeric species expressed in step (a) in vitro to form active dimers,
at least one of the monomeric species being OP1.

22. The process of claim 20 or claim 21 wherein the heterodimeric protein comprises an OP1/BMP2 heterodimer.

23. Active osteogenic protein composition comprising a polypeptide chain consisting essentially of any one of the polypeptides OP1-16V, OP1-16L, OP1-16M and OP1-16A, optionally further comprising OP1-18.

24. Active osteogenic composition according to claim 23 comprising any one of the polypeptides OP1-16V, OP1-16L, OP1-16M and OP1-16A dimerized with OP1-18.

25. The polypeptide of claim 1, composition of any one of claims 2, 23 and 24, device of claim 3 or protein of claim 18 or 19 wherein the protein or polypeptide is unglycosylated.

26. An osteogenic device for implantation in a mammal, the device comprising:
(a) a biocompatible, in vivo biodegradable matrix competent to serve as a scaffold for the anchoring and proliferation of recruited progenitor cells;
(b) the polypeptide of claim 1 or claim 25, composition of any one of claims 2, 23 and 24, device of claim 3, OP1 of claim 16 or protein of claim 11,17, 18 or 19.

27. The device of claim 26 wherein said matrix comprises: (a) allogenic or xenogenic bone; or (b) a collagen matrix in the form of an encapsulated powder or shape-retaining interadherent particles.

28. The device of claim 26 or 27 as dependent on claim 23 or 24 wherein said matrix comprises demineralized, delipidated, Type I insoluble bone collagen particles, depleted in noncollagenous protein, and treated with a hot aqueous medium having a temperature above about 37 degrees centigrade in an amount and for a time sufficient to alter the morphology of said particles.

29. The device of claim 28 wherein said matrix is treated with a hot aqueous medium having a temperature within the range of 37 to 65 (for example 45 to 60) degrees centigrade.

30. The device of claim 28 or claim 29 wherein said matrix is treated to increase the number of pores and micropits on said collagen particles at least 3-fold, e.g. at least 10-fold.

31. The device of any one of claims 28-30 wherein said bone collagen particles comprise pores or micropits having a mean diameter within the range of 1µm to 100µm.

32. The device of any one of claims 28-31 wherein said collagen particles have a mean diameter within the range of 70mm to 420mm.

33. The device of any one of claims 26-32 comprising demineralized, delipidated, Type-I insoluble bone collagen particles, depleted in a material comprising fraction 12 identified in Figure 8.

34. The device of any one of claims 26-33 for use in therapy, e.g. for bone formation, filling a bone defect, periodontal treatment, cartilage repair, the treatment of osteoarthritis, the correction of non-union fractures, the treatment of acquired or congenital craniofacial and other skeletal or dental anomalies.

## Patentansprüche

1. Isoliertes Präpro- oder Pro-OP1-Polypeptid.

2. Zusammensetzung, die das isolierte Polypeptid nach Anspruch 1 umfaßt.

3. Osteogenetisches Mittel mit dem isolierten Polypeptid nach Anspruch 1 oder der Zusammensetzung nach Anspruch 2, die in einer Matrix dispergiert sind, wobei das Polypeptid geeignet dimerisiert und gefaltet ist.

4. Isolierte DNA, die Präpro- oder Pro-OP1 kodiert.

5. DNA nach Anspruch 4,
wobei die DNA umfaßt:
(a) die den Positionen 49-1341 von Seq. ID Nr. 7 (Fig. 2) entsprechende Nukleotid-Sequenz; oder
(b) eine Sequenz, die die den Resten 1-431 von Seq. ID Nr. 7 (Fig. 2) entsprechende Aminosäuresequenz kodiert; oder
(c) eine DNA-Sequenz, die ein mutiertes Präpro- oder Pro-OP1-Protein kodiert.

6. DNA nach Anspruch 5,
wobei die DNA die zu den Positionen 1-1822 von Seq. ID Nr. 7 (Fig. 2) entsprechende Nukleotid-Sequenz umfaßt.

7. DNA nach einem der Ansprüche 4 bis 6,
die cDNA, genomische DNA oder DNA ist, die aus Oligonukleotiden aufgebaut ist.

8. Vektor mit der DNA nach einem der Ansprüche 4 bis 7 in operativer Verbindung mit einer Sequenz zur Expressionskontrolle.

9. Wirtszelle, die mit dem Vektor nach Anspruch 8 oder der DNA nach einem der Ansprüche 5-7 transformiert ist.

10. Wirtszelle nach Anspruch 9, die prokaryotisch oder eukaryotisch, beispielsweise eine Säugetier-, Saccharomyces- oder E. coli-Zelle ist.

11. Osteogenetisches Protein, das durch Expression aus einer Säugetier-Wirtszelle, die mit der DNA nach Anspruch 5 transformiert ist, erzeugt ist, und die dazu befähigt ist, die Knorpel- und Knochenbildung bei einem Säugetier zu induzieren.

12. Verfahren zur Erzeugung eines osteogenetischen Proteins, das dazu in der Lage ist, die Knorpel- und Knochenbildung bei einem Säugetier zu induzieren, wobei das Verfahren die folgenden Schritte umfaßt:
(a) Kultivieren der Wirtszelle nach Anspruch 9 oder 10 in einem geeigneten Kulturmedium, und
(b) Wiedergewinnen, Isolieren und Reinigen dieses Proteins aus diesem Kulturmedium.

13. Verfahren zur Erzeugung von OP1-Protein mit dem Schritt der Kultivierung einer Zelle, die Pro- oder Präpro-OP1 kodierende DNA enthält, unter derartigen Bedingungen, daß diese DNA zur Herstellung eines OP1-Proteins exprimiert wird, das durch post-translationale, proteolytische Spaltung und Absonderung aus der Zelle einer Reifung unterworfen ist.

14. Verfahren nach Anspruch 13, bei dem die Zelle eine Säugetier-Wirtszelle ist, wobei die Wirtszelle in einem Kulturmedium kultiviert wird, und die DNA die Aminosäuren 1 bis 431 von Sequenz ID Nr. 7 (oder eine dazu verwandte oder daraus mutierte Aminosäure-Sequenz) kodiert, und das weiterhin den Schritt der Wiedergewinnung dieses Proteins aus diesem Kulturmedium umfaßt.

15. Verfahren zur Erzeugung einer aktiven osteogenetischen Protein-Zusammensetzung, das den Schritt der Verkürzung des reifen OP1 umfaßt.

16. Reifes OP1, wie es aus einer Säugetier-Wirtszellen-Kultur bei oder nach Expression der in Fig. 2 dargestellten Sequenz abgesondert wird.

17. Osteogenetisches Protein, das dazu fähig ist, die Knorpel- und Knochenbildung bei einem Säugetier zu induzieren, das durch die folgenden Schritte gewinnbar ist:
(a) Kultivieren einer Säugetier-Wirtszelle, die mit einer, die Aminosäuren 1 bis 431 von Sequenz ID Nr. 7 (oder eine dazu verwandte oder daraus mutierte DNA-Sequenz) kodierenden, DNA-Sequenz transformiert ist, in einer geeigneten Kultur, und
(b) Wiedergewinnen dieses Proteins aus diesem Kulturmedium.

18. Aktives osteogenetisches Protein, das ein OP1/BMP2 Heterodimer umfaßt.

19. Protein nach Anspruch 18, bei dem das Heterodimer durch Disulfid-Brücken verbunden ist oder anders gebunden ist.

20. Verfahren zur Erzeugung einer Zusammensetzung, die ein aktives heterodimeres, osteogenetisches Protein umfaßt, mit dem Schritt, zwei oder mehr monomere Spezies' in einer eukaryotischen Zelle (beispielsweise einer Säugetierzelle) zu falten und zu oxidieren, wobei zumindest eine der monomeren Spezies' OP1 ist.

21. Verfahren zur Erzeugung einer Zusammensetzung mit einem aktiven heterodimeren, osteogenetischen Protein, mit den Schritten:
(a) Expression einer einzelnen monomeren Spezies; und
(b) Oxidieren und Rückfaltung jeder der in Schritt (a) exprimierten monomeren Spezies' in vitro, um aktive Dimere zu bilden,
wobei zumindest eine der monomeren Spezies' OP1 ist.

22. Verfahren nach Anspruch 20 oder Anspruch 21, bei dem das heterodimere Protein ein OP1/BMP2-Heterodimer umfaßt.

23. Aktive osteogenetische Protein-Zusammensetzung mit einer Polypeptidkette, die im wesentlichen aus einem der Polypeptide OP1-16V, OP1-16L, OP1-16M und OP1-16A besteht, und die weiterhin optional OP1-18 umfaßt.

24. Aktive osteogenetische Zusammensetzung nach Anspruch 23, mit einem der Polypeptide OP1-16V, OP1-16L, OP1-16M und OP1-16A, das mit OP1-18 dimerisiert ist.

25. Polypeptid nach Anspruch 1, Zusammensetzung nach einem der Ansprüche 2, 23 und 24, Mittel nach Anspruch 3 oder Protein nach Anspruch 18 oder 19, wobei das Protein oder das Polypeptid unglykosiliert ist.

26. Osteogenetische Mittel zur Implantation in ein Säugetier, wobei das Mittel folgendes umfaßt:
(a) eine biokompatible, in vivo biologisch-abbaubare Matrix, die dazu in der Lage ist als ein Gerüst zur Verankerung und Proliferation von herangezogenen Vorläuferzellen zu dienen;
(b) das Polypeptid nach Anspruch 1 oder Anspruch 25, die Zusammensetzung nach einem der Ansprüche 2, 23 und 24, das Mittel nach Anspruch 3, OP1 nach Anspruch 16 oder das Protein nach Anspruch 11, 17, 18 oder 19.

27. Mittel nach Anspruch 26, bei dem die Matrix folgendes umfaßt:
(a) allogenetische oder xenogenetische Knochen; oder
(b) eine Kollagen-Matrix in Form eines verkapselten Pulvers oder von Form-beständigen, aneinanderhaftenden Partikeln.

28. Mittel nach Anspruch 26 oder 27, unter Rückbeziehung auf Anspruch 23 oder 24, bei dem die Matrix demineralisierte, entfettete, unlösliche Typ-I-Knochen-Kollagen-Partikel umfaßt, die in nicht-kollagenem Protein abgereichert wurden und die mit einem Heißwasser-Medium mit einer Temperatur oberhalb ungefähr 37°C in einer Menge und für eine Zeit behandelt wurden, die ausreicht, um die Morphologie dieser Partikel zu verändern.

29. Mittel nach Anspruch 28, bei dem die Matrix mit einem Heißwasser-Medium mit einer Temperatur im Bereich von 37 bis 65 (beispielsweise 45 bis 60) Grad Celsius behandelt wird.

30. Mittel nach Anspruch 28 oder Anspruch 29, bei dem die Matrix behandelt ist, um die Anzahl von Poren und Mikroporen auf diesen Kollagen-Partikeln zumindest um das 3-fache, beispielsweise zumindest um das 10-fache zu vergrößern.

31. Mittel nach einem der Ansprüche 28 bis 30, bei dem die Knochen-Kollagen-Partikel Poren oder Mikroporen mit einem mittleren Durchmesser im Bereich von 1 µm bis 100 µm umfassen.

32. Mittel nach einem der Ansprüche 28 bis 31, bei dem diese Kollagen-Partikel einen mittleren Durchmesser im Bereich von 70 mm bis 420 mm aufweisen.

33. Mittel nach einem der Ansprüche 26 bis 32, mit demineralisierten, entfetteten, unlöslichen Typ-I-Knochen-Kollagen-Partikeln, die in einem Material, das die Fraktion 12 umfaßt, die in Fig. 8 identifiziert wurde, abgereichert wurden.

34. Medium nach einem der Ansprüche 26 bis 33, zur Verwendung bei einer Therapie, beispielsweise zur KnochenBildung, zur Auffüllung eines Knochendefekts, für eine Periodontal-Behandlung, für die Knorpel-Wiederherstellung, für die Behandlung der Osteoarthritis, zur Korrektur der Pseudoarthrose, zur Behandlung von erworbenen oder angeborenen kraniofacialen- und anderen Skelett- oder Zahn-Anomalien.

## Revendications

1. Polypeptide prépro- ou pro-OP1 isolé.

2. Composition comprenant le polypeptide isolé selon la revendication 1.

3. Dispositif ostéogène comprenant le polypeptide isolé selon la revendication 1 ou la composition selon la revendication 2, dispersé dans une matrice, le polypeptide étant correctement dimérisé et plié.

4. ADN isolé encodant prépro- ou pro-OP1.

5. ADN selon la revendication 4, ledit ADN comprenant:
(a) la séquence nucléotidique correspondant aux positions 49-1341 de Seq. ID. No. 7 (figure 2); ou
(b) une séquence encodant la séquence d'acides aminés correspondant aux résidus 1-431 de Seq. ID. No. 7, figure 2; ou
(c) une séquence d'ADN encodant une mutéine prépro- ou pro-OP1.

6. ADN selon la revendication 5, ledit ADN comprenant la séquence nucléotidique correspondant aux positions 1-1822 de Seq. ID. No. 7, figure 2.

7. ADN selon l'une quelconque des revendications 4 à 6, à savoir de l'ADNc, de l'ADN génomique ou de l'ADN construit à partir d'oligonucléotides.

8. Vecteur comprenant l'ADN selon l'une quelconque des revendications 4 à 7, en association d'entraînement avec une séquence de commande d'expression pour ce dernier.

9. Cellule hôte transformée avec le vecteur selon la revendication 8 ou avec l'ADN selon l'une quelconque des revendications 5 à 7.

10. Cellule hôte selon la revendication 9, à savoir une cellule procaryote ou eucaryote, par exemple une cellule de mammifère, une cellule Saccharomyces ou une cellule E. coli.

11. Protéine ostéogène produite par expression à partir d'une cellule hôte de mammifère transformée avec l'ADN selon la revendication 5 et compétente pour induire une formation de cartilage et d'os chez un mammifère.

12. Procédé pour produire une protéine ostéogène capable d'induire une formation de cartilage et d'os chez un mammifère, le procédé comprenant les étapes consistant à:
(a) cultiver dans un milieu de culture approprié, la cellule hôte selon la revendication 9 ou 10; et
(b) récupérer, isoler et purifier ladite protéine à partir dudit milieu de culture.

13. Procédé pour produire la protéine OP1, comprenant l'étape consistant à cultiver une cellule contenant de l'ADN encodant pro- ou prépro-OP1 dans des conditions telles que ledit ADN est exprimé pour produire une protéine OP1 qui est soumise à une maturation par sécrétion et clivage protéolytique post-traductionnels à partir de la cellule.

14. Procédé selon la revendication 13, dans lequel la cellule est une cellule hôte de mammifère, la cellule hôte est cultivée dans un milieu de culture et l'ADN encode les acides aminés 1 à 431 de la séquence ID. No. 7 (ou encore une séquence d'acides aminés apparentée à cette dernière ou obtenue par mutation à partir de cette dernière), comprenant en outre l'étape consistant à récupérer ladite protéine dudit milieu de culture.

15. Procédé pour produire une composition protéinique ostéogène active, comprenant l'étape consistant à tronquer la OP1 mûre.

16. OP1 mûre telle que sécrétée à partir d'une culture de cellules hôtes de mammifères au cours de ou après l'expression de la séquence représentée en figure 2.

17. Protéine ostéogène compétente pour induire une formation de cartilage et d'os chez un mammifère, que l'on obtient en passant par les étapes consistant à:
(a) cultiver dans une culture appropriée, une cellule hôte de mammifère transformée avec une séquence d'ADN encodant les acides aminés 1 à 431 de la séquence ID. No. 7 (ou encore une séquence d'ADN apparentée à cette dernière ou obtenue par mutation de cette dernière), et
(b) récupérer ladite protéine dudit milieu de culture.

18. Protéine ostéogène active comprenant un hétérodimère OP1/BMP2.

19. Protéine selon la revendication 18, dans laquelle l'hétérodimère est joint par des liaisons disulfures ou associé d'une autre manière.

20. Procédé pour produire une compostion comprenant une protéine ostéogène hétérodimère active, comprenant l'étape consistant à plier et à oxyder deux espèces monomères ou plus dans une cellule eucaryote (par exemple une cellule de mammifère), au moins une des espèces monomères étant OP1.

21. Procédé pour produire une composition comprenant une protéine ostéogène hétérodimère active, comprenant les étapes consistant à:
(a) exprimer des espèces monomères individuelles; et
(b) oxyder et replier chacune des espèces monomères exprimées à l'étape (a) in vitro pour former des dimères actifs,
au moins une des espèces monomères étant OP1.

22. Procédé selon la revendication 20 ou 21, dans lequel la protéine hétérodimère comprend un hétérodimère OP1/BMP2.

23. Composition protéinique ostéogène active comprenant une chaîne polypeptidique constituée essentiellement par l'un quelconque des polypeptides OP1-16V, OP1-16L, OP1-16M et OP1-16A, comprenant, en outre, le cas échéant, OP1-18.

24. Composition ostéogène active selon la revendication 23, comprenant l'un quelconque des polypeptides OP1-16V, OP1-16L, OP1-16M et OP1-16A, dimérisé avec OP1-18.

25. Polypeptide selon la revendication 1, composition selon l'une quelconque des revendications 2, 23 et 24, dispositif selon la revendication 3, ou encore protéine selon la revendication 18 ou 19, dans lequel la protéine ou le polypeptide est non glycosylé.

26. Dispositif ostéogène pour une implantation dans un mammifère, le dispositif comprenant:
(a) une matrice biocompatible, biodégradable in vivo, compétente pour servir de support pour l'ancrage et la prolifération de cellules souches recrutées;
(b) le polypeptide selon la revendication 1 ou selon la revendication 25, la composition selon l'une quelconque des revendications 2, 23 et 24, le dispositif selon la revendication 3, l'OP1 selon la revendication 16 ou encore la protéine selon la revendication 11, 17, 18 ou 19.

27. Dispositif selon la revendication 26, dans lequel ladite matrice comprend: (a) un os allogène ou xénogène; ou (b) une matrice collagène sous la forme d'une poudre encapsulée ou de particules interadhérentes résistant à la déformation.

28. Dispositif selon la revendication 26 ou 27 lorsqu'elles dépendent de la revendication 23 ou 24, dans lequel ladite matrice comprend des particules de collagène osseux insoluble de type I, délipidées, déminéralisées, est dépourvue de protéine non collagène et est traitée avec un milieu aqueux chaud possédant une température supérieure à environ 37 degrés centigrades, en une quantité et pendant un laps de temps suffisants pour modifier la morphologie desdites particules.

29. Dispositif selon la revendication 28, dans lequel ladite matrice est traitée avec un milieu aqueux chaud dont la température se situe dans le domaine de 37 à 65 (par exemple de 45 à 60) degrés centigrades.

30. Dispositif selon la revendication 28 ou selon la revendication 29, dans lequel ladite matrice est traitée pour augmenter le nombre de pores et de microtrous sur lesdites particules de collagène à concurrence d'au moins 3 fois, par exemple d'au moins 10 fois.

31. Dispositif selon l'une quelconque des revendications 28 à 30, dans lequel lesdites particules de collagène osseux comprennent des pores ou des microtrous dont le diamètre moyen se situe dans le domaine de 1 µm à 100 µm.

32. Dispositif selon l'une quelconque des revendications 28 à 31, dans lequel lesdites particules de collagène possèdent un diamètre moyen qui se situe dans le domaine de 70 mm à 420 mm.

33. Dispositif selon l'une quelconque des revendications 26 à 32, comprenant des particules de collagène osseux insoluble de type I, délipidées, déminéralisées, dépourvu d'une matière comprenant la fraction 12 identifiée en figure 8.

34. Dispositif selon l'une quelconque des revendications 26 à 33, à utiliser en thérapie, par exemple pour l'ostéogenèse, pour le remplissage d'une lacune osseuse, pour le traitement périodontique, pour la réparation de cartilage, pour le traitement de l'arthrose, pour la correction d'absence de soudure d'un os fracturé, pour le traitement d'anomalies acquises ou congénitales de type craniofacial et d'autres anomalies squelettiques ou dentaires.
